(19) Europäisches Patentamt
European Patent Office
Office européen des brevets

(11) **EP 3 932 303 B1**

(12) **EUROPEAN PATENT SPECIFICATION**

(45) Date of publication and mention
of the grant of the patent:
**23.10.2024 Bulletin 2024/43**

(21) Application number: **20773834.5**

(22) Date of filing: **11.01.2020**

(51) International Patent Classification (IPC):
*A61B 5/18* (2006.01)     *A61B 5/16* (2006.01)
*A61B 5/291* (2021.01)     *A61B 5/372* (2021.01)
*A61B 5/00* (2006.01)     *G16H 40/67* (2018.01)
*G16H 50/30* (2018.01)     *G16H 50/20* (2018.01)
*G16H 20/70* (2018.01)

(52) Cooperative Patent Classification (CPC):
**A61B 5/372; A61B 5/168; A61B 5/18; A61B 5/291;
A61B 5/6817; A61B 5/6843; A61B 5/7203;
A61B 5/7267; A61B 5/7278; A61B 5/7285;
G16H 20/70; G16H 40/67; G16H 50/20;
G16H 50/30**

(86) International application number:
**PCT/CN2020/071565**

(87) International publication number:
**WO 2020/186915 (24.09.2020 Gazette 2020/39)**

(54) **METHOD AND SYSTEM FOR DETECTING ATTENTION**

VERFAHREN UND SYSTEM ZUR DETEKTION VON AUFMERKSAMKEIT

PROCÉDÉ ET SYSTÈME DE DÉTECTION DE L'ATTENTION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **15.03.2019 CN 201910199425**

(43) Date of publication of application:
**05.01.2022 Bulletin 2022/01**

(73) Proprietor: **Huawei Technologies Co., Ltd.
Shenzhen, Guangdong 518129 (CN)**

(72) Inventors:
• **NI, Gang**
  **Shenzhen, Guangdong 518129 (CN)**
• **YANG, Hui**
  **Shenzhen, Guangdong 518129 (CN)**
• **ZHA, Jun**
  **Shenzhen, Guangdong 518129 (CN)**
• **TANG, Weidong**
  **Shenzhen, Guangdong 518129 (CN)**
• **LI, Hao**
  **Shenzhen, Guangdong 518129 (CN)**

(74) Representative: **Goddar, Heinz J.
Boehmert & Boehmert
Anwaltspartnerschaft mbB
Pettenkoferstrasse 22
80336 München (DE)**

(56) References cited:
**WO-A1-2016/110804     WO-A1-2017/146956
CN-A- 101 836 859     CN-A- 101 987 017
CN-A- 103 610 447     CN-A- 103 610 447
CN-A- 103 942 568     CN-A- 104 814 735
CN-A- 105 982 665     CN-A- 106 371 610
CN-A- 106 667 484     CN-A- 106 919 948
CN-A- 107 280 663     CN-A- 109 471 528
CN-A- 110 584 657**

• **NAKAMURA TAKASHI ET AL: "Automatic
detection of drowsiness using in-ear EEG", 2018
INTERNATIONAL JOINT CONFERENCE ON
NEURAL NETWORKS (IJCNN), IEEE, 8 July 2018
(2018-07-08), pages 1 - 6, XP033419569, DOI:
10.1109/IJCNN.2018.8489723**

**(Cont. next page)**

- SEUNGHYEOK HONG ET AL: "Intelligent system for drowsiness recognition based on ear canal electroencephalography with photoplethysmography and electrocardiography", INFORMATION SCIENCES, vol. 453, 1 July 2018 (2018-07-01), AMSTERDAM, NL, pages 302 - 322, XP055558066, ISSN: 0020-0255, DOI: 10.1016/j.ins.2018.04.003
- HA UNSOO ET AL: "A multimodal drowsiness monitoring ear-module system with closed-loop real-time alarm", 2016 IEEE BIOMEDICAL CIRCUITS AND SYSTEMS CONFERENCE (BIOCAS), IEEE, 17 October 2016 (2016-10-17), pages 536 - 539, XP033051130, DOI: 10.1109/BIOCAS.2016.7833850

## Description

## TECHNICAL FIELD

This application relates to the data processing field, and in particular, to a method and a system for detecting attention of a driver during safe driving and assisted driving.

## BACKGROUND

With the development of society and the popularization of automobiles, safe driving has become one of important concerns of traffic safety assurances. A status of a driver is one of important factors affecting safe driving. Inattentive driving includes any driving activity that distracts attention of a driver, such as taking a car, eating and drinking, talking with a passenger, adjusting an entertainment system or a navigation system, and making a call, and is also related to a mental status or several consciousness changes of the driver. For example, the driver is in a near-sleep state for a short time due to fatigue. Studies have shown that up to 30% of traffic accidents are caused because drivers are inattentive. When a vehicle is traveling at a relatively high speed, if a distracted driver cannot be fully aware of real-time changes in statuses of a path, traffic, an obstacle, and even the vehicle, an accident will inevitably occur.

In L1 and L2 self-driving, a driver is responsible for a driving process and vehicle control all the time. Therefore, for traffic safety assurances, it is quite important to use a vehicle system to accurately and promptly detect and analyze a status of the driver and assist in providing an alert at appropriate time when attention of the driver is not focused.

In the prior art, a driving attention type of a driver is determined by using an intelligent computer system and based on signals collected by an automobile system, such as a fixation point, a line of sight, a rest time, and saccades of the driver, and movement statuses of surrounding objects along a driving path. In such a technology, various sensors, a driving computer system, and the like usually need to be installed in the automobile system, leading to relatively high costs. Due to complexity and diversity of driving environments, there is a specific probability of deviation in a driving attention status of the driver obtained through intelligent calculation, affecting safe driving.

In recent years, with the rapid development of electroencephalogram signal collection and analysis technologies, a vehicle system accurately determines a driving attention type of a driver by collecting an electroencephalogram EEG signal of the driver, to accurately and promptly detect and analyze a status of the driver and assist in providing an alert for a driving behavior of the driver. This provides another effective technology implementation option for traffic safety assurances.

However, how to obtain an electroencephalogram signal of a driver more conveniently and accurately and how to accurately determine a status of the driver by using the electroencephalogram signal have become research focuses in safe driving.

NAKAMURA TAKASHI et al disclose the "automatic detection of drowsiness using in-ear EEG" in 2018 published at the INTERNATIONAL JOINT CONFERENCE ON NEURAL NETWORKS (IJCNN), IEEE, (20180708), doi: 10. 1 109/IJCNN.2018.8489723.

WO 2016/110804 A1 discloses one or more wearable devices i.e., attached or applied to limbs, body, head or other body extremities but also applicable to implanted or physiologically attachable systems. These systems have a means of enabling diagnostic or prognostic monitoring applicable to monitoring relevant parameters and corresponding analysis determination and characterization applicable to the onset or detection of events or health conditions of interest. One application relates to sleep monitoring and associate EEG sensors.

## SUMMARY

The invention is defined by independent claims 1, 9 and 13. Alternative embodiments are defined by their respective dependent claims. Embodiments of this application provide an attention detection method and system, to perform attention detection on a driver. An electroencephalogram signal is obtained from an ear side, so that it is more convenient and feasible to obtain the electroencephalogram signal during driving. This reduces measurement costs, and can ensure accuracy of obtaining the electroencephalogram signal.

According to a first aspect, an embodiment provides a user attention detection method, as defined by independent claim 1. The method includes: collecting a user bioelectrical signal from an ear side of a user by using an ear-side wearing apparatus; obtaining a user electroencephalogram signal from the user bioelectrical signal; and obtaining an attention type of the user based on the user electroencephalogram signal and a machine learning model. The ear-side wearing apparatus comprises a plurality of ear-side signal measurement units and the collecting a user bioelectrical signal from an ear side of a user by using an ear-side wearing apparatus specifically includes: determining whether an impedance between two of the ear-side signal measurement units is less than a preset threshold, collecting bioelectrical signals from the two ear-side signal measurement units when the impedance between the two ear-side signal measurement units is less than the preset threshold, obtaining the user bioelectrical signal based on a potential difference signal corresponding to the bioelectrical signals collected by the two ear-side signal measurement units, And determining, based on an impedance value between ear-side signal measurement units, whether the ear-side signal measurement units is attached to skin, so as to select different signal collection policies depending on different cases.

In the foregoing method, it is more convenient and faster to obtain the electroencephalogram signal from the ear side, and the ear-side wearing apparatus is convenient to carry, is disposed on the ear side, and is not easy to fall off during wearing, so that it is more convenient and feasible to measure the user electroencephalogram signal during driving. In addition, based on characteristics of the bioelectrical signals collected from the ear side, the collected user bioelectrical signal is processed through potential difference processing. This can effectively remove noise in the electroencephalogram signal. Moreover, considering that the user possibly does not wear the ear-side wearing apparatus correctly, a device on one side of the ear-side wearing apparatus is faulty, or signal receiving performed by the ear-side wearing apparatus is undesirable, determining needs to be performed on the collected bioelectrical signals before the potential difference processing is performed, so as to avoid an inaccurate result caused because signal collection and processing are still performed when the ear-side wearing apparatus cannot normally perform receiving.

In some implementations of the first aspect, the plurality of ear-side signal measurement units include a left-ear-side signal measurement unit and a right-ear-side signal measurement unit, and the determining whether an impedance between two of the ear-side signal measurement units is less than a preset threshold, collecting bioelectrical signals from the two ear-side signal measurement units when the impedance between the two ear-side signal measurement units is less than the preset threshold, and obtaining the user bioelectrical signal based on a potential difference signal corresponding to the bioelectrical signals collected by the two ear-side signal measurement units is specifically:
determining whether an impedance between the left-ear-side signal measurement unit and the right-ear-side signal measurement unit is less than a preset threshold, and when the impedance between the left-ear-side signal measurement unit and the right-ear-side signal measurement unit is less than the preset threshold, obtaining the user bioelectrical signal based on a potential difference signal corresponding to a bioelectrical signal measured by the left-ear-side signal measurement unit and a bioelectrical signal measured by the right-ear-side signal measurement unit.

In other words, the ear-side wearing apparatus may obtain the bioelectrical signals from both left and right ear sides; and when it is determined that the ear-side wearing apparatus is normally worn on both the sides, obtain the user bioelectrical signal based on the bioelectrical signals obtained from the left and right ears.

In some implementations of the first aspect, the ear-side wearing apparatus is a single-ear-side wearing apparatus, the plurality of ear-side signal measurement units include a plurality of single-ear-side signal measurement units, and the determining whether an impedance between two of the ear-side signal measurement units is less than a preset threshold, collecting bioelectrical signals from the two ear-side signal measurement units when the impedance between the two ear-side signal measurement units is less than the preset threshold, and obtaining the user bioelectrical signal based on a potential difference signal corresponding to the bioelectrical signals collected by the two ear-side signal measurement units is specifically:
determining whether an impedance between two of the single-ear-side signal measurement units is less than a preset threshold, and when the impedance between the two single-ear-side signal measurement units is less than the preset threshold, obtaining the user bioelectrical signal based on a potential difference signal corresponding to bioelectrical signals collected by the two single-ear-side signal measurement units.

According to the foregoing manner, the ear-side wearing apparatus may be a single-ear wearing apparatus, and whether the ear-side wearing apparatus is normally worn on a single ear is directly determined based on an impedance between two measurement units.

In some implementations of the first aspect, the method includes: when there are a plurality of left-ear-side signal measurement units, and there are a plurality of right-ear-side signal measurement units, and when an impedance between one of the left-ear-side signal measurement units and one of the right-ear-side signal measurement units is greater than the preset threshold, determining whether an impedance between two of the plurality of left-ear-side signal measurement units is less than a preset threshold and whether an impedance between two of the plurality of right-ear-side signal measurement units is less than a preset threshold; and obtaining the user bioelectrical signal based on a potential difference signal corresponding to bioelectrical signals measured by two bioelectrical measurement apparatuses that are on one ear canal side and between which an impedance is less than the preset threshold.

According to the foregoing manner, if an electroencephalogram signal can be obtained from only one ear canal side, whether the ear-side wearing apparatus is normally worn on one side may further be determined; and if the ear-side wearing apparatus is normally worn on one side, in the foregoing implementation, the user bioelectrical signal can still be correctly obtained.

In some implementations of the first aspect, the obtaining the user bioelectrical signal based on a potential difference signal corresponding to the bioelectrical signals collected by the two ear-side signal measurement units is specifically: obtaining, by using a differential circuit, the potential difference signal corresponding to the bioelectrical signals collected by the two ear-side signal measurement units, and using the potential difference signal as the user bioelectrical signal.

In some implementations of the first aspect, the obtaining an attention type of the user based on the user electroencephalogram signal and a machine learning model is specifically: calculating a sample entropy value of the user electroencephalogram signal, and analyzing the attention type of the user based on the sample entropy value and the machine learning model.

In some implementations of the first aspect, the detecting an attention type of the user based on the user electroencephalogram signal is specifically: intercepting the user electroencephalogram signal of a preset time length, and obtaining N signal sampling points from the user electroencephalogram signal of the preset time length, where the N signal sampling points are u(1), u(2), ..., and u(N); sequentially intercepting m sampling points based on the N signal sampling points by using each of u(1), u(2), ..., and u(N-m+1) as a start point, to construct N-m+1 m-dimensional vectors; calculating, for each of the N-m+1 m-dimensional vectors, a ratio of a quantity of vectors that are in all the other vectors and whose distances to the m-dimensional vector are less than r to a quantity of all the other vectors, and calculating an average value of the obtained N-m+1 ratios to obtain a first average value; sequentially intercepting m+1 sampling points based on the N signal sampling points by using each of u(1), u(2), ..., and u(N-m) as a start point, to construct N-m (m+1)-dimensional vectors; calculating, for each of the N-m (m+1)-dimensional vectors, a ratio of a quantity of vectors that are in all the other vectors and whose distances to the (m+1)-dimensional vector are less than r to a quantity of all the other vectors, and calculating an average value of the obtained N-m ratios to obtain a second average value; and calculating a sample entropy (SampEn) value based on a ratio of the first average value to the second average value.

In some implementations of the first aspect, the machine learning model is an SVM classifier; and machine learning is performed by using the SVM classifier, to obtain a segmentation value, and the attention type of the user is determined based on the segmentation value and the sample entropy value.

According to the foregoing manner, the attention type of the user is obtained based on the sample entropy value and the machine learning model. In this way, through machine learning, sample entropy characteristics of electroencephalogram signals corresponding to different attention types can be obtained through analysis more accurately, so as to determine a current attention type of the user based on a sample entropy value of a collected electroencephalogram signal.

According to a second aspect, an embodiment provides a user attention detection system, as defined by independent claim 9. The system includes: an ear-side wearing apparatus, configured to: collect a user bioelectrical signal from an ear side of a user, and obtain a user electroencephalogram signal from the user bioelectrical signal; and obtain an attention detection apparatus, configured to detect an attention type of the user based on the user electroencephalogram signal. That the ear-side wearing apparatus comprises a plurality of ear-side signal measurement units, and the ear-side wearing apparatus is configured to collect the user bioelectrical signal from the ear side of the user specifically includes: the ear-side wearing apparatus determines whether an impedance between two of the ear-side signal measurement units is less than a preset threshold, collects bioelectrical signals from the two ear-side signal measurement units when the impedance between the two ear-side signal measurement units is less than the preset threshold, obtains the user bioelectrical signal based on a potential difference signal corresponding to the bioelectrical signals collected by the two ear-side signal measurement units, and determines, based on an impedance value between ear-side signal measurement units, whether the ear-side signal measurement units is attached to skin, so as to select different signal collection policies depending on different cases.

In some implementations of the second aspect, the plurality of ear-side signal measurement units include a left-ear-side signal measurement unit and a right-ear-side signal measurement unit, and that the ear-side wearing apparatus determines whether an impedance between two of the ear-side signal measurement units is less than a preset threshold, collects bioelectrical signals from the two ear-side signal measurement units when the impedance between the two ear-side signal measurement units is less than the preset threshold, and obtains the user bioelectrical signal based on a potential difference signal corresponding to the bioelectrical signals collected by the two ear-side signal measurement units is specifically: The ear-side wearing apparatus determines whether an impedance between the left-ear-side signal measurement unit and the right-ear-side signal measurement unit is less than a preset threshold, and when the impedance between the left-ear-side signal measurement unit and the right-ear-side signal measurement unit is less than the preset threshold, obtains the user bioelectrical signal based on a potential difference signal corresponding to a bioelectrical signal measured by the left-ear-side signal measurement unit and a bioelectrical signal measured by the right-ear-side signal measurement unit.

In some implementations of the second aspect, the ear-side wearing apparatus is a single-ear-side wearing apparatus, the single-ear-side wearing apparatus includes a plurality of single-ear-side signal measurement units, and that the ear-side wearing apparatus determines whether an impedance between two of the ear-side signal measurement units is less than a preset threshold, collects bioelectrical signals from the two ear-side signal measurement units when the impedance between the two ear-side signal measurement units is less than the preset threshold, and obtains the user bioelectrical signal based on a potential difference signal corresponding to the bioelectrical signals collected by the two ear-side signal measurement units is specifically: The ear-side wearing apparatus determines whether an impedance between two of the single-ear-side signal measurement units is less than a preset threshold, and when the impedance between the two single-ear-side signal measurement units is less than the preset threshold, obtains the user bioelectrical signal based on a potential difference signal corresponding to bioelectrical signals collected by the two single-ear-side signal measurement units.

In some implementations of the second aspect, there are a plurality of left-ear-side signal measurement units; there are a plurality of right-ear-side signal measurement units; and when an impedance between one of the left-ear-side signal measurement units and one of the right-ear-side signal measurement units is greater than the preset threshold, the ear-side wearing apparatus determines whether an impedance between two of the plurality of left-ear-side signal measurement units is less than a preset threshold and whether an impedance between two of the plurality of right-ear-side signal measurement units is less than a preset threshold, and obtains the user bioelectrical signal based on a potential difference signal corresponding to bioelectrical signals measured by two bioelectrical measurement apparatuses that are on one ear canal side and between which an impedance is less than the preset threshold.

In some implementations of the second aspect, that the attention detection apparatus obtains the attention type of the user based on the user electroencephalogram signal and a machine learning model is specifically: The attention detection apparatus calculates a sample entropy value of the user electroencephalogram signal, and analyzes the attention type of the user based on the sample entropy value and the machine learning model.

According to a third aspect, an embodiment provides an ear-side wearing apparatus, defined by independent claim 13. The apparatus includes: a plurality of ear-side signal measurement units, configured to collect a user bioelectrical signal from an ear side; a first determining unit, configured to: determine whether an impedance between two of the ear-side signal measurement units is less than a preset threshold, when the impedance between the two ear-side signal measurement units is less than the preset threshold, use a potential difference signal corresponding to bioelectrical signals collected and measured by the two ear-side signal measurement units as the user bioelectrical signal, and determine, based on an impedance value between ear-side signal measurement units, whether the ear-side signal measurement units is attached to skin, so as to select different signal collection policies depending on different case; a characteristic decomposition unit, configured to obtain an electroencephalogram signal from the user bioelectrical signal; and an attention detection unit, configured to obtain an attention type of a user based on the electroencephalogram signal and a machine learning model.

In some implementations of the third aspect, the plurality of ear-side signal measurement units include a left-ear-side signal measurement unit and a right-ear-side signal measurement unit; and the first determining unit is configured to: determine whether an impedance between the left-ear-side signal measurement unit and the right-ear-side signal measurement unit is less than a preset threshold, and when the impedance between the left-ear-side signal measurement unit and the right-ear-side signal measurement unit is less than the preset threshold, obtain the user bioelectrical signal based on a potential difference signal corresponding to a bioelectrical signal measured by the left-ear-side signal measurement unit and a bioelectrical signal measured by the right-ear-side signal measurement unit.

In some implementations of the third aspect, the ear-side wearing apparatus is a single-ear-side wearing apparatus, the plurality of ear-side signal measurement units include a plurality of single-ear-side signal measurement units, and that the first determining unit is configured to: determine whether an impedance between two of the ear-side signal measurement units is less than a preset threshold, and when the impedance between the two ear-side signal measurement units is less than the preset threshold, use a potential difference signal corresponding to bioelectrical signals collected and measured by the two ear-side signal measurement units as the user bioelectrical signal is specifically: The first determining unit is configured to: determine whether an impedance between two of the single-ear-side signal measurement units is less than a preset threshold, and when the impedance between the two single-ear-side signal measurement units is less than the preset threshold, use a potential difference signal corresponding to bioelectrical signals collected by the two single-ear-side signal measurement units as the user bioelectrical signal.

In some implementations of the third aspect, there are a plurality of left-ear-side signal measurement units; there are a plurality of right-ear-side signal measurement units; and the ear-side wearing apparatus further includes a second determining unit. When the first determining unit determines that an impedance between one of the left-ear-side signal measurement units and one of the right-ear-side signal measurement units is greater than the preset threshold, the second determining unit determines whether an impedance between two of the plurality of left-ear-side signal measurement units less than a preset threshold and whether an impedance between two of the plurality of right-ear-side signal measurement units is less than a preset threshold, and uses a potential difference signal corresponding to bioelectrical signals collected by two bioelectrical measurement apparatuses that are on one ear canal side and between which an impedance is less than the preset threshold as the user bioelectrical signal.

In some implementations of the third aspect, that the attention detection unit obtains the attention type of the user based on the electroencephalogram signal and the machine learning model is specifically: The attention detection unit calculates a sample entropy value of the user electroencephalogram signal, and analyzes the attention type of the user based on the sample entropy value and the machine learning model.

According to a fourth aspect, an embodiment provides an ear-side wearing apparatus. The apparatus includes: a plurality of ear-side signal measurement units, configured to collect a user bioelectrical signal from an ear side; a first determining unit, configured to: determine whether an impedance between two of the ear-side signal measurement units is less than a preset threshold, when the impedance between the two ear-side signal measurement units is less than the preset threshold, use a potential difference signal corresponding to bioelectrical signals collected and measured by the two ear-side signal measurement units as the user bioelectrical signal, and determine, based on an impedance value between ear-side signal measurement units, whether the ear-side signal measurement units is attached to skin, so as to select different signal collection policies depending on different case; a characteristic decomposition unit, configured to obtain an electroencephalogram signal from the user bioelectrical signal; and a sending unit, configured to send the electroencephalogram signal to a signal analysis apparatus.

In some implementations of the fourth aspect, the plurality of ear-side signal measurement units include a left-ear-side signal measurement unit and a right-ear-side signal measurement unit; and the first determining unit is configured to: determine whether an impedance between the left-ear-side signal measurement unit and the right-ear-side signal measurement unit is less than a preset threshold, and when the impedance between the left-ear-side signal measurement unit and the right-ear-side signal measurement unit is less than the preset threshold, obtain the user bioelectrical signal based on a potential difference signal corresponding to a bioelectrical signal measured by the left-ear-side signal measurement unit and a bioelectrical signal measured by the right-ear-side signal measurement unit.

In some implementations of the fourth aspect, the ear-side wearing apparatus is a single-ear-side wearing apparatus, the plurality of ear-side signal measurement units include a plurality of single-ear-side signal measurement units, and that the first determining unit is configured to: determine whether an impedance between two of the ear-side signal measurement units is less than a preset threshold, and when the impedance between the two ear-side signal measurement units is less than the preset threshold, use a potential difference signal corresponding to bioelectrical signals collected and measured by the two ear-side signal measurement units as the user bioelectrical signal is specifically: The first determining unit is configured to: determine whether an impedance between two of the single-ear-side signal measurement units is less than a preset threshold, and when the impedance between the two single-ear-side signal measurement units is less than the preset threshold, use a potential difference signal corresponding to bioelectrical signals collected by the two single-ear-side signal measurement units as the user bioelectrical signal.

In some implementations of the fourth aspect, there are a plurality of left-ear-side signal measurement units; there are a plurality of right-ear-side signal measurement units; and the ear-side wearing apparatus further includes a second determining unit. When the first determining unit determines that an impedance between one of the left-ear-side signal measurement units and one of the right-ear-side signal measurement units is greater than the preset threshold, the second determining unit determines whether an impedance between two of the plurality of left-ear-side signal measurement units is less than a preset threshold and whether an impedance between two of the plurality of right-ear-side signal measurement units is less than a preset threshold, and uses a potential difference signal corresponding to bioelectrical signals collected by two bioelectrical measurement apparatuses that are on one ear canal side and between which an impedance is less than the preset threshold as the user bioelectrical signal.

According to a fifth aspect, an embodiment provides an attention detection apparatus. The apparatus includes: a receiving unit, configured to receive a user electroencephalogram signal from an ear-side wearing apparatus; and an attention detection unit, configured to obtain an attention type of a user based on the user electroencephalogram signal and a machine learning model.

In some implementations of the fifth aspect, the attention detection unit is specifically configured to: calculate a sample entropy value of the user electroencephalogram signal, and analyze the attention type of the user based on the sample entropy value and the machine learning model.

In some implementations of the fifth aspect, the attention detection unit is specifically configured to: intercept the user electroencephalogram signal of a preset time length, and obtain N signal sampling points from the user electroencephalogram signal of the preset time length, where the N signal sampling points are $u(1)$, $u(2)$, ..., and $u(N)$; sequentially intercept m sampling points based on the N signal sampling points by using each of $u(1)$, $u(2)$, ..., and $u(N-m+1)$ as a start point, to construct $N-m+1$ m-dimensional vectors; calculate, for each of the $N-m+1$ m-dimensional vectors, a ratio of a quantity of vectors that are in all the other vectors and whose distances to the m-dimensional vector are less than r to a quantity of all the other vectors, and calculate an average value of the obtained $N-m+1$ ratios to obtain a first average value; sequentially intercept m+1 sampling points based on the N signal sampling points by using each of $u(1)$, $u(2)$, ..., and $u(N-m)$ as a start point, to construct $N-m$ (m+1)-dimensional vectors; calculate, for each of the $N-m$ (m+1)-dimensional vectors, a ratio of a quantity of vectors that are in all the other vectors and whose distances to the (m+1)-dimensional vector are less than r to a quantity of all the other vectors, and calculate an average value of the obtained N-m ratios to obtain a second average value; and calculate a sample entropy (SampEn) value based on a ratio of the first average value to the second average value.

In some implementations of the fifth aspect, the machine learning model is an SVM classifier; machine learning is performed by using the SVM classifier, to obtain a segmentation value; and the attention detection unit determines the attention type of the user based on the segmentation value and the sample entropy value.

According to a sixth aspect, an embodiment provides an ear-side wearing apparatus. The apparatus includes: a plurality of ear-side signal measurement units, configured to collect a user bioelectrical signal from an ear side; a processor, configured to: determine whether an impedance between two of the ear-side signal measurement units is less than a preset threshold, and when the impedance between the two ear-side signal measurement units is less than the preset threshold, use a potential difference signal corresponding to bioelectrical signals collected and measured by the two ear-side signal measurement units as the user bioelectrical signal; a characteristic decomposition unit, configured to obtain an electroencephalogram signal from the user bioelectrical signal; and an attention detection unit, configured to obtain an attention type of a user based on the electroencephalogram signal and a machine learning model.

In some implementations of the sixth aspect, the plurality of ear-side signal measurement units include a left-ear-side signal measurement unit and a right-ear-side signal measurement unit; and the processor is configured to: determine whether an impedance between the left-ear-side signal measurement unit and the right-ear-side signal measurement unit is less than a preset threshold, when the impedance between the left-ear-side signal measurement unit and the right-ear-side signal measurement unit is less than the preset threshold, obtain the user bioelectrical signal based on a potential difference signal corresponding to a bioelectrical signal measured by the left-ear-side signal measurement unit and a bioelectrical signal measured by the right-ear-side signal measurement unit, and determine, based on an impedance value between ear-side signal measurement units, whether the ear-side signal measurement units is attached to skin, so as to select different signal collection policies depending on different cases.

In some implementations of the sixth aspect, the ear-side wearing apparatus is a single-ear-side wearing apparatus, the plurality of ear-side signal measurement units include a plurality of single-ear-side signal measurement units, and that the processor is configured to: determine whether an impedance between two of the ear-side signal measurement units is less than a preset threshold, and when the impedance between the two ear-side signal measurement units is less than the preset threshold, use a potential difference signal corresponding to bioelectrical signals collected and measured by the two ear-side signal measurement units as the user bioelectrical signal is specifically: The processor is configured to: determine whether an impedance between two of the single-ear-side signal measurement units is less than a preset threshold, and when the impedance between the two single-ear-side signal measurement units is less than the preset threshold, use a potential difference signal corresponding to bioelectrical signals collected by the two single-ear-side signal measurement units as the user bioelectrical signal.

In some implementations of the sixth aspect, there are a plurality of left-ear-side signal measurement units; there are a plurality of right-ear-side signal measurement units; and the processor is further configured to: when the first determining unit determines that an impedance between one of the left-ear-side signal measurement units and one of the right-ear-side signal measurement units is greater than the preset threshold, determine whether an impedance between two of the plurality of left-ear-side signal measurement units is less than a preset threshold and whether an impedance between two of the plurality of right-ear-side signal measurement units is less than a preset threshold, and use a potential difference signal corresponding to bioelectrical signals collected by two bioelectrical measurement apparatuses that are on one ear canal side and between which an impedance is less than the preset threshold as the user bioelectrical signal.

In some implementations of the sixth aspect, that the attention detection unit obtains the attention type of the user based on the electroencephalogram signal and the machine learning model is specifically: The attention detection unit calculates a sample entropy value of the user electroencephalogram signal, and analyzes the attention type of the user based on the sample entropy value and the machine learning model.

According to a seventh aspect, an embodiment provides an ear-side wearing apparatus. The apparatus includes: a plurality of ear-side signal measurement units, configured to collect a user bioelectrical signal from an ear side; a processor, configured to: determine whether an impedance between two of the ear-side signal measurement units is less than a preset threshold, when the impedance between the two ear-side signal measurement units is less than the preset threshold, use a potential difference signal corresponding to bioelectrical signals collected and measured by the two ear-side signal measurement units as the user bioelectrical signal, and determine, based on an impedance value between ear-side signal measurement units, whether the ear-side signal measurement units is attached to skin, so as to select different signal collection policies depending on different cases; a characteristic decomposition unit, configured to obtain an electroencephalogram signal from the user bioelectrical signal; and a sending unit, configured to send the electroencephalogram signal to a signal analysis apparatus.

In some implementations of the seventh aspect, the plurality of ear-side signal measurement units include a left-ear-side signal measurement unit and a right-ear-side signal measurement unit; and the processor is configured to: determine whether an impedance between the left-ear-side signal measurement unit and the right-ear-side signal measurement unit is less than a preset threshold, and when the impedance between the left-ear-side signal measurement unit and the right-ear-side signal measurement unit is less than the preset threshold, obtain the user bioelectrical signal based on a potential difference signal corresponding to a bioelectrical signal measured by the left-ear-side signal measurement unit and a bioelectrical signal measured by the right-ear-side signal measurement unit.

In some implementations of the seventh aspect, the ear-side wearing apparatus is a single-ear-side wearing apparatus, the plurality of ear-side signal measurement units include a plurality of single-ear-side signal measurement units, and that the processor determines whether an impedance between two of the ear-side signal measurement units is less than a preset threshold, and when the impedance between the two ear-side signal measurement units is less than the preset threshold, uses a potential difference signal corresponding to bioelectrical signals collected and measured by the two ear-side signal measurement units as the user bioelectrical signal is specifically: The processor determines whether an impedance between two of the single-ear-side signal measurement units is less than a preset threshold, and when the impedance between the two single-ear-side signal measurement units is less than the preset threshold, uses a potential difference signal corresponding to bioelectrical signals collected by the two single-ear-side signal measurement units as the user bioelectrical signal.

In some implementations of the seventh aspect, there are a plurality of left-ear-side signal measurement units; there are a plurality of right-ear-side signal measurement units; and the processor is further configured to: when the first determining unit determines that an impedance between one of the left-ear-side signal measurement units and one of the right-ear-side signal measurement units is greater than the preset threshold, determine whether an impedance between two of the plurality of left-ear-side signal measurement units is less than a preset threshold and whether an impedance between two of the plurality of right-ear-side signal measurement units is less than a preset threshold, and use a potential difference signal corresponding to bioelectrical signals collected by two bioelectrical measurement apparatuses that are on one ear canal side and between which an impedance is less than the preset threshold as the user bioelectrical signal.

According to an eighth aspect, an embodiment provides an attention detection apparatus. The apparatus includes: a receiving unit, configured to receive a user electroencephalogram signal from an ear-side wearing apparatus; and a processor, configured to obtain an attention type of a user based on the user electroencephalogram signal and a machine learning model.

In some implementations of the eighth aspect, the processor is specifically configured to: calculate a sample entropy value of the user electroencephalogram signal, and analyze the attention type of the user based on the sample entropy value and the machine learning model.

In some implementations of the eighth aspect, the processor is specifically configured to: intercept the user electroencephalogram signal of a preset time length, and obtain N signal sampling points from the user electroencephalogram signal of the preset time length, where the N signal sampling points are u(1), u(2), ..., and u(N); sequentially intercept m sampling points based on the N signal sampling points by using each of u(1), u(2), ..., and u(N-m+1) as a start point, to construct N-m+1 m-dimensional vectors; calculate, for each of the N-m+1 m-dimensional vectors, a ratio of a quantity of vectors that are in all the other vectors and whose distances to the m-dimensional vector are less than r to a quantity of all the other vectors, and calculate an average value of the obtained N-m+1 ratios to obtain a first average value; sequentially intercept m+1 sampling points based on the N signal sampling points by using each of u(1), u(2), ..., and u(N-m) as a start point, to construct N-m (m+1)-dimensional vectors; calculate, for each of the N-m (m+1)-dimensional vectors, a ratio of a quantity of vectors that are in all the other vectors and whose distances to the (m+1)-dimensional vector are less than r to a quantity of all the other vectors, and calculate an average value of the obtained N-m ratios to obtain a second average value; and calculate a sample entropy (SampEn) value based on a ratio of the first average value to the second average value.

In some implementations of the eighth aspect, the machine learning model is an SVM classifier; machine learning is performed by using the SVM classifier, to obtain a segmentation value; and the attention detection unit determines the attention type of the user based on the segmentation value and the sample entropy value.

According to a ninth aspect, an embodiment provides an electroencephalogram signal detection method. The method includes: collecting a user bioelectrical signal from an ear side by using a plurality of ear-side signal measurement units; determining whether an impedance between two of the ear-side signal measurement units is less than a preset threshold; when the impedance between the two ear-side signal measurement units is less than the preset threshold, using a potential difference signal corresponding to bioelectrical signals collected and measured by the two ear-side signal measurement units as the user bioelectrical signal, and determining, based on an impedance value between ear-side signal measurement units (111, 121, 1402), whether the ear-side signal measurement units (111, 121, 1402) is attached to skin, so as to select different signal collection policies depending on different cases; obtaining an electroencephalogram signal from the user bioelectrical signal; and sending the electroencephalogram signal to a signal analysis apparatus.

In some implementations of the ninth aspect, the plurality of ear-side signal measurement units include a left-ear-side signal measurement unit and a right-ear-side signal measurement unit; and the determining step is specifically: determining whether an impedance between the left-ear-side signal measurement unit and the right-ear-side signal measurement unit is less than a preset threshold, and when the impedance between the left-ear-side signal measurement unit and the right-ear-side signal measurement unit is less than the preset threshold, obtaining the user bioelectrical signal based on a potential difference signal corresponding to a bioelectrical signal measured by the left-ear-side signal measurement unit and a bioelectrical signal measured by the right-ear-side signal measurement unit.

In some implementations of the ninth aspect, the ear-side wearing apparatus is a single-ear-side wearing apparatus, and the plurality of ear-side signal measurement units include a plurality of single-ear-side signal measurement units; and the determining step is specifically: determining whether an impedance between two of the single-ear-side signal measurement units is less than a preset threshold, and when the impedance between the two single-ear-side signal measurement units is less than the preset threshold, using, a potential difference signal corresponding to bioelectrical signals collected by the two single-ear-side signal measurement units as the user bioelectrical signal.

In some implementations of the ninth aspect, the method includes: when there are a plurality of left-ear-side signal measurement units, and there are a plurality of right-ear-side signal measurement units, and when the first determining unit determines that an impedance between one of the left-ear-side signal measurement units and one of the right-ear-side signal measurement units is greater than the preset threshold, determining whether an impedance between two of the plurality of left-ear-side signal measurement units is less than a preset threshold and whether an impedance between two of the plurality of right-ear-side signal measurement units is less than a preset threshold, and using a potential difference signal corresponding to bioelectrical signals collected by two bioelectrical measurement apparatuses that are on one ear canal side and between which an impedance is less than the preset threshold as the user bioelectrical signal.

According to a tenth aspect, an embodiment provides an attention detection method. The method includes: receiving a user electroencephalogram signal from an ear-side wearing apparatus; and obtaining an attention type of a user based on the user electroencephalogram signal and a machine learning model.

In some implementations of the tenth aspect, the obtaining an attention type of a user based on the user electroencephalogram signal and a machine learning model is specifically: calculating a sample entropy value of the user electroencephalogram signal, and analyzing the attention type of the user based on the sample entropy value and the machine learning model.

In some implementations of the tenth aspect, the calculating a sample entropy value of the user electroencephalogram signal is specifically: intercepting the user electroencephalogram signal of a preset time length, and obtaining N signal sampling points from the user electroencephalogram signal of the preset time length, where the N signal sampling points are u(1), u(2), ..., and u(N); sequentially intercepting m sampling points based on the N signal sampling points by using each of u(1), u(2), ..., and u(N-m+1) as a start point, to construct N-m+1 m-dimensional vectors; calculating, for each of the N-m+1 m-dimensional vectors, a ratio of a quantity of vectors that are in all the other vectors and whose distances to the m-dimensional vector are less than r to a quantity of all the other vectors, and calculating an average value of the obtained N-m+1 ratios to obtain a first average value; sequentially intercepting m+1 sampling points based on the N signal sampling points by using each of u(1), u(2), ..., and u(N-m) as a start point, to construct N-m (m+1)-dimensional vectors; calculating, for each of the N-m (m+1)-dimensional vectors, a ratio of a quantity of vectors that are in all the other vectors and whose distances to the (m+1)-dimensional vector are less than r to a quantity of all the other vectors, and calculating an average value of the obtained N-m ratios to obtain a second average value; and calculating a sample entropy (SampEn) value based on a ratio of the first average value to the second average value.

In some implementations of the tenth aspect, the machine learning model is an SVM classifier; machine learning is performed by using the SVM classifier, to obtain a segmentation value; and the attention detection unit determines the attention type of the user based on the segmentation value and the sample entropy value.

In some implementations of the foregoing aspects, the ear-side wearing apparatus is an earplug or an earphone.

In some implementations of the foregoing aspects, the attention type of the user may specifically be that attention of the user is in a focused state or a distracted state.

In some implementations of the foregoing aspects, the plurality of ear-side signal measurement units mean two or more ear-side signal measurement units.

In some implementations of the foregoing aspects, the determining whether an impedance between two of the ear-side signal measurement units is less than a preset threshold may be: selecting two ear-side signal measurement units from the plurality of ear-side signal measurement units based on a preset setting to perform comparison; or selecting two ear-side signal measurement units based on a specified priority sequence to perform comparison, and when an impedance between two ear-side signal measurement units selected each time is less than the preset threshold, terminating the comparison after the comparison is performed for a preset quantity of times or all comparison operations are completed.

In some implementations of the foregoing aspects, the determining whether an impedance between the left-ear-side signal measurement unit and the right-ear-side signal measurement unit is less than a preset threshold may be: when there is one left-ear-side signal measurement unit and one right-ear-side signal measurement unit, directly performing comparison; or when there are a plurality of left-ear-side signal measurement units and a plurality of right-ear-side signal measurement units, selecting two left-ear-side signal measurement units from the plurality of left-ear-side signal measurement units and two right-ear-side signal measurement units from the plurality of right-ear-side signal measurement units based on a preset setting to perform comparison, or separately selecting two left-ear-side signal measurement units and two right-ear-side signal measurement units based on a specified priority sequence to perform comparison, and when an impedance between two ear-side signal measurement units selected each time is less than the preset threshold, terminating the comparison after the comparison is performed for a preset quantity of times or all comparison operations are completed.

In some implementations of the foregoing aspects, determining whether an impedance between the plurality of single-ear-side signal measurement units is less than the preset threshold may be: when there are two single-ear-side signal measurement units, directly performing comparison; or selecting two single-ear-side signal measurement units from the plurality of single-ear-side signal measurement units based on a preset setting to perform comparison, or selecting two ear-side signal measurement units based on a specified priority sequence to perform comparison, and when an impedance between two ear-side signal measurement units selected each time is less than the preset threshold, terminating the comparison after the comparison is performed for a preset quantity of times or all comparison operations are completed.

In some implementations of the foregoing aspects, the determining whether an impedance between two of the plurality of left-ear-side signal measurement units is less than a preset threshold and whether an impedance between two of the plurality of right-ear-side signal measurement units is less than a preset threshold, when it is determined that an impedance between one of the left-ear-side signal measurement units and one of the right-ear-side signal measurement units is greater than the preset threshold may be: when there are two left-ear-side signal measurement units and two right-ear-side signal measurement units, directly performing comparison for the two left-ear-side signal measurement units and for the two right-ear-side signal measurement units; or separately selecting two left-ear-side signal measurement units from the plurality of left-ear-side signal measurement units and two right-ear-side signal measurement units from the plurality of right-ear-side signal measurement units based on a preset setting to perform comparison; or for the left-ear-side signal measurement units, selecting two left-ear-side signal measurement units based on a specified priority sequence to perform comparison, and when an impedance between two left-ear-side signal measurement units selected each time is less than the preset threshold, terminating the comparison after the comparison is performed for a preset quantity of times or all comparison operations are completed, and for the right-ear-side signal measurement units, selecting two right-ear-side signal measurement units based on a specified priority sequence to perform comparison, and when an impedance between two right-ear-side signal measurement units selected each time is less than the preset threshold, terminating the comparison after the comparison is performed for a preset quantity of times or all comparison operations are completed.

It can be learnt that, by implementing the technical solutions in the embodiments of this application, a prior-art problem that it is inconvenient to perform attention determining and it is prone to cause an inaccurate attention determining result in a moving state can be resolved. The electroencephalogram signal of the driver is collected from an ear, so that it is more convenient and feasible to collect the user electroencephalogram signal. In addition, due to an electrode attachment requirement, in this solution, whether the ear-side wearing apparatus is normally worn currently can be determined, and it is ensured that the ear-side wearing apparatus performs signal collection and subsequent analysis when the ear-side wearing apparatus can normally perform collection, thereby ensuring accuracy of a detection result. In addition, electroencephalogram signals collected from left and right ear canals are processed through potential difference processing, so as to ensure accuracy of the collected electroencephalogram signals; and sample entropies of the collected and processed electroencephalogram signals are calculated to obtain electroencephalogram signals that are consistent in time domain, and attention determining is performed by using an SVM classification algorithm. In this way, a current driving attention type of the driver can be relatively accurately determined, so as to accurately provide a subsequent operation during driving, for example, providing an alert to the driver or performing a corresponding emergency operation. The invention is defined by the appended claims.

**BRIEF DESCRIPTION OF DRAWINGS**

To describe the technical solutions in the embodiments of this application or in the background more clearly, the following briefly describes the accompanying drawings required for describing the embodiments of this application or the background.

FIG. 1 is a schematic diagram of an application scenario according to an embodiment of this application;
FIG. 2a is a schematic flowchart of a user attention detection method according to an embodiment of this application;
FIG. 2b is a schematic flowchart of detecting, in a process of obtaining a user electroencephalogram signal, whether an ear-side wearing apparatus is normally worn according to an embodiment of this application;

FIG. 2c is a schematic flowchart of detecting, in a process of obtaining a user electroencephalogram signal, whether a single-ear-side wearing apparatus is normally worn according to an embodiment of this application;

FIG. 3 is a schematic diagram of $\alpha$, $\beta$, $\gamma$, $\theta$, and $\delta$ brain waveforms generated by a brain;

FIG. 4 is a schematic flowchart of a user attention detection method according to an embodiment of this application;

FIG. 5 shows an implementation of a differential circuit in a method for obtaining a user electroencephalogram signal according to an embodiment of this application;

FIG. 6 is a differential processing principle diagram of electroencephalogram signals from left and right ears in an attention detection method according to an embodiment of this application;

FIG. 7 is a schematic differential processing diagram of electroencephalogram signals from left and right ears in an attention detection method according to an embodiment of this application;

FIG. 8a shows a muscle artifact generated by a neck joint action;

FIG. 8b shows an ocular artifact generated by winking;

FIG. 9a is a schematic principle diagram of SVM classification;

FIG. 9b is a schematic principle diagram of SVM classification;

FIG. 10 is a schematic structural diagram of an attention detection system according to an embodiment of this application;

FIG. 11a is a schematic structural diagram of an ear-side wearing apparatus according to an embodiment of this application;

FIG. 11b is a schematic structural diagram of another ear-side wearing apparatus according to an embodiment of this application;

FIG. 11c is a schematic structural diagram of another ear-side wearing apparatus according to an embodiment of this application;

FIG. 11d is a schematic structural diagram of another ear-side wearing apparatus according to an embodiment of this application;

FIG. 12 is a schematic diagram of a specific implementation form of an ear-side wearing apparatus according to an embodiment of this application;

FIG. 13 is a schematic diagram of positions at which an ear-side wearing apparatus is worn according to an embodiment of this application;

FIG. 14 is a schematic structural diagram of an attention detection apparatus according to an embodiment of this application;

FIG. 15a is a schematic structural diagram of an ear-side wearing apparatus according to an embodiment of this application;

FIG. 15b is a schematic structural diagram of an attention analysis apparatus according to an embodiment of this application;

FIG. 16 is a flowchart of a method for measuring a user-related signal according to an embodiment of this application; and

FIG. 17 is a flowchart of an attention detection method according to an embodiment of this application.

**DESCRIPTION OF EMBODIMENTS**

The following describes implementations of this application by using examples with reference to the accompanying drawings in the embodiments of this application. However, implementations of this application may further include a combination of the embodiments as far as they are encompassed by the appended claims. Therefore, the detailed description of the following embodiments should not be understood in a restrictive sense. Terms used in the embodiments of this application are merely used to describe specific embodiments of this application, but are not intended to limit this application, while the invention is defined by the appended claims.

One or more structural compositions of functions, modules, features, units, and the like mentioned in specific embodiments of this application can be understood as being implemented in any manner by using any physical or tangible component (for example, software, hardware (such as a logical function implemented by a processor or chip), and/or any other combination running on a computer device). In some embodiments, different modules or units obtained through division from various devices shown in the accompanying drawings may reflect the use of corresponding different physical and tangible components in actual implementations. Optionally, a single module in the accompanying drawings in the embodiments of this application may be implemented by using a plurality of actual physical components. Similarly, any two or more modules depicted in the accompanying drawings may reflect different functions performed by a single actual physical component.

For flowcharts of a method in the embodiments of this application, some operations are described as different steps performed in a specific sequence. Such flowcharts are used for illustrative purposes rather than restrictive purposes. Some steps described in this specification may be combined and performed in a single operation, some steps may be divided into a plurality of substeps, and some steps may be performed in a sequence different from that shown in this specification. The steps shown in the flowchart may be implemented in any manner by using any circuit structure and/or tangible mechanism (for example, software, hardware (such as a logical function implemented by a processor or chip), and/or any combination thereof running on a computer device).

In the following descriptions, one or more features may be identified as "optional". This type of statement should not be construed as an exhaustive indication of features that may be considered as optional. In other words, although not explicitly identified in this specification, other features may be considered as optional. In addition, any description of a single entity is not intended to exclude the use of a plurality of such entities. Similarly, a description of a plurality of entities is not intended to exclude the use of a single entity. Finally, the term "for example" refers to one of many potential implementations.

The embodiments of this application are mainly used for user attention detection, and may specifically be applied to attention detection of a driver during driving, to determine whether attention of the driver is focused, so that an alert can be provided in time based on a determining result. In addition, the embodiments of this application may also be applied to another scenario in which user attention detection needs to be performed.

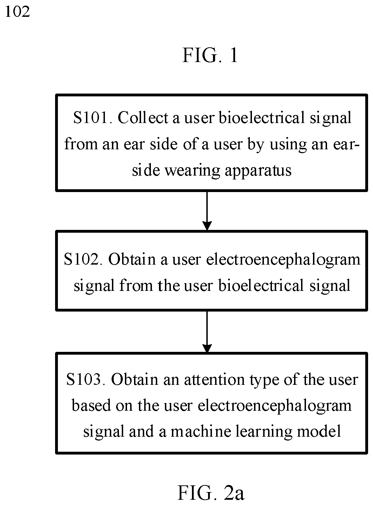

FIG. 1 is a typical application scenario according to an embodiment of the present disclosure. An ear-side wearing apparatus 101 (which may specifically be an earphone or an earplug) is worn on an ear of a user, collects a bioelectrical signal of the driver from an ear side, and sends the bioelectrical signal of the driver to a user attention detection apparatus 102. Specific operations of the ear-side wearing apparatus 101 may optionally further include: collecting bioelectrical signals from the ear side by using ear-side signal measurement units; obtaining a potential difference between the bioelectrical signals collected by the measurement units, to perform signal enhancement and eliminate interference from an external cluttered interference signal; and performing artifact removal processing, filtering out a non-electroencephalogram frequency signal (for example, filtering out a waveform whose frequency is greater than 32 Hz) by using a filter circuit, and extracting a waveform characteristic through wavelet analysis, for subsequent digital coding. The attention detection apparatus 102 (which may specifically be a handheld terminal such as a mobile phone, a PDA, or a pad, or a vehicle-mounted terminal device) analyzes a user electroencephalogram signal. When discovering, through determining, that attention is distracted, the attention detection apparatus 102 performs a corresponding subsequent operation, such as providing an alert to a driver in time by using an alarm device, to ensure driving safety. An attention analysis manner may be calculating a sample entropy value of an electroencephalogram signal and performing classification on the sample entropy by using an SVN algorithm to determine an attention status.

In addition, to ensure that the ear-side wearing apparatus 101 can collect an accurate bioelectrical signal, the ear-side wearing apparatus 101 further performs pre-determining on whether the ear-side wearing apparatus 101 can normally collect a signal, and determines, based on an impedance value between ear-side signal measurement units, whether the ear-side signal measurement units is attached to skin, so as to select different signal collection policies depending on different cases.

The ear side in this embodiment of the present disclosure refers to an area that is on and near an ear of a human body and in which a bioelectrical signal can be measured, for example, positions on an inner side of an ear canal, on an auricle, in an auricular groove, at a back of the ear, and around the ear. Ear-side signal measurement units are deployed in an area on an ear of a human body and near the ear of the human body to collect bioelectrical signals.

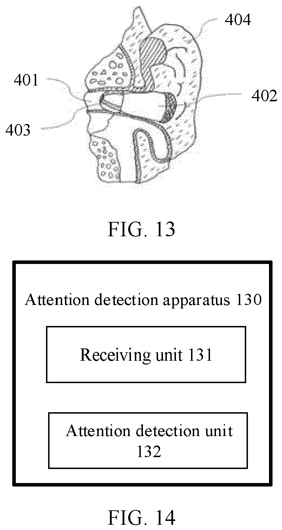

FIG. 13 shows an example wearing manner, that is, a signal collection manner, of an ear-side wearing apparatus according to an embodiment of the present disclosure. An example of a signal collection manner of obtaining a bioelectrical signal from an inner side of an ear canal is provided. 401 represents an ear canal of a human body, 403 represents an ear-side signal measurement unit, 402 represents a main body of the ear-side wearing apparatus, and 404 represents an auricle of a user.

FIG. 2a is a schematic flowchart of a method for obtaining a user electroencephalogram signal according to an embodiment of this application. A specific procedure includes the following steps.

S101. Collect a user bioelectrical signal from an ear side of a user by using an ear-side wearing apparatus.

S101 specifically includes: After the ear-side wearing apparatus is worn, enable an electroencephalogram signal collection function of the device, and collect the user electroencephalogram signal from the ear side by using the ear-side wearing apparatus. A wearing manner has been described above, and details are not described herein again. There may be a plurality of manners of enabling the device. The ear-side wearing apparatus may be enabled to enter a working state, by pressing a physical button on an earphone, or through triggering by using a corresponding APP on a user attention detection apparatus (which may be a mobile phone, a vehicle-mounted terminal, or the like) (for example, by touching a virtual button for starting driving in the APP).

The ear-side wearing apparatus may fall off or may not be correctly worn during wearing. Therefore, when a signal collected by the ear-side wearing apparatus is directly obtained for processing, a measurement result may be inaccurate because the device falls off or is not correctly worn. As a result, an attention type of the user cannot be correctly analyzed. Therefore, in this embodiment of this application, a wearing status of the ear-side wearing apparatus is determined, and whether data is to be collected or whether collected data is used to analyze the attention type of the user is determined based on a determining result.

S101 specifically includes: When the ear-side wearing apparatus includes a plurality of ear-side signal measurement units, determine whether an impedance between two of the ear-side signal measurement units is less than a preset threshold; and when the impedance between the two ear-side signal measurement units is less than the preset threshold, use a potential difference signal corresponding to bioelectrical signals measured by the two ear-side signal measurement units as the user bioelectrical signal.

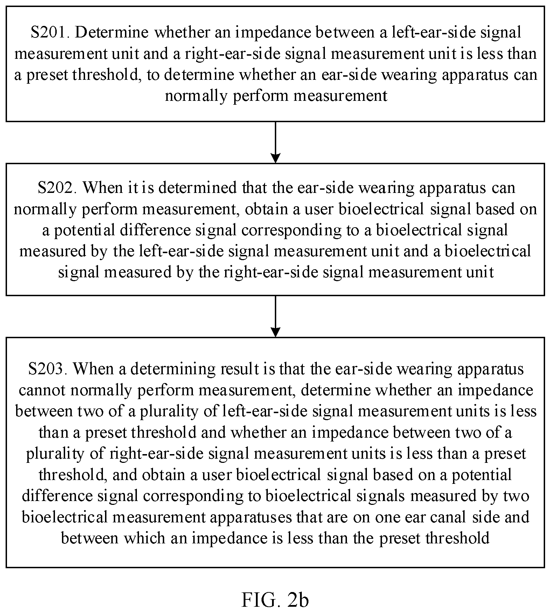

Corresponding to a case in which the ear-side wearing apparatus is a dual-side measurement apparatus, that is, the ear-side wearing apparatus includes a left-ear-side signal measurement unit and a right-ear-side signal measurement unit, and a manner of collecting a bioelectrical signal from the ear side may further be shown in FIG. 2b, and includes the following steps.

S201. Determine whether an impedance between the left-ear-side signal measurement unit and the right-ear-side signal measurement unit is less than a preset threshold, to determine whether the ear-side wearing apparatus can normally perform measurement.

Whether the impedance between the left-ear-side signal measurement unit and the right-ear-side signal measurement unit is less than the preset threshold is determined to determine whether the ear-side wearing apparatus can normally perform measurement (that is, can be normally worn).

Specifically, there may be one or more left-ear-side signal measurement units and one or more right-ear-side signal measurement units. In an implementation process of the left/right-ear-side signal measurement unit, the left/right-ear-side signal measurement unit may be in a form of an electrode, and the user bioelectrical signal on the ear side is measured by using the electrode. The impedance value between the left-ear-side signal measurement unit and the right-ear-side signal measurement unit is determined to determine whether the left-ear-side signal measurement unit and the right-ear-side signal measurement unit of the ear-side wearing apparatus are attached to ear canals, that is, whether the ear-side wearing apparatus is correctly worn. When the left-ear-side signal measurement unit and the right-ear-side signal measurement unit are attached to the ear canals, the impedance value between the left-ear-side signal measurement unit and the right-ear-side signal measurement unit is relatively small and is usually less than an impedance value of a surface of the ear side. When either or neither of the left-ear-side signal measurement unit and the right-ear-side signal measurement unit is attached to the ear canals, the impedance value between the left-ear-side signal measurement unit and the right-ear-side signal measurement unit is relatively large and is usually greater than the impedance value of the surface of the ear side. Therefore, a preset threshold may be set to determine a wearing status of the ear-side wearing apparatus. Optionally, the preset impedance determining threshold may be the impedance value of the surface of the ear side.

When there is one left-ear-side signal measurement unit and one right-ear-side signal measurement unit, an impedance value between the two measurement units is directly obtained for determining.

When there are a plurality of left-ear-side signal measurement units and a plurality of right-ear-side signal measurement units, there may be a plurality of measurement policies. For example, one left-ear-side signal measurement unit and one right-ear-side signal measurement unit are arbitrarily selected to obtain an impedance value between the two measurement units; or an impedance value between a left-ear-side signal measurement unit and a right-ear-side signal measurement units at preset positions is obtained only once, and whether the ear-side wearing apparatus is normally worn on left and right ears is determined based on the obtained impedance value. Alternatively, a priority sequence may be set to perform measurement by matching measurement unit pairs one by one, and when the preset threshold is not satisfied, measurement and determining are terminated after measurement is performed for a preset quantity of times. Alternatively, measurement is performed on measurement unit pairs one by one until it is learnt, through measurement, that an impedance between one pair of measurement units is less than the preset value. In this case, it indicates that the ear-side wearing apparatus can normally perform measurement; or otherwise, when it is learnt, through measurement, that an impedance between any pair of measurement units is not less than the preset value, it indicates that the ear-side wearing apparatus cannot work normally. A specific measurement method performed in a case in which there are a plurality of measurement units is not limited herein.

5202. When it is determined that the ear-side wearing apparatus can normally perform measurement, obtain the user bioelectrical signal based on a potential difference signal corresponding to a bioelectrical signal collected by the left-ear-side signal measurement unit and a bioelectrical signal collected by the right-ear-side signal measurement unit.

In other words, when the impedance between the left-ear-side signal measurement unit and the right-ear-side signal measurement unit is less than the preset threshold, the user bioelectrical signal is obtained based on the potential difference signal corresponding to the bioelectrical signal collected by the left-ear-side signal measurement unit and the bioelectrical signal collected by the right-ear-side signal measurement unit.

Specifically, when there is one left-ear-side signal measurement unit and one right-ear-side signal measurement unit, when it is learnt, through measurement, that an impedance between the left-ear-side signal measurement unit and the right-ear-side signal measurement unit is less than the preset threshold, it is determined that the ear-side wearing apparatus can normally perform measurement, and the user bioelectrical signal is obtained based on a potential difference signal corresponding to a bioelectrical signal collected by the left-ear-side signal measurement unit and a bioelectrical signal collected by the right-ear-side signal measurement unit.

A specific manner of obtaining the user bioelectrical signal based on the potential difference signal corresponding to the bioelectrical signal collected by the left-ear-side signal measurement unit and the bioelectrical signal collected by the right-ear-side signal measurement unit may include: directly using the potential difference signal corresponding to the bioelectrical signal collected by the left-ear-side signal measurement unit and the bioelectrical signal collected by the right-ear-side signal measurement unit as the user bioelectrical signal; or configuring a reference electrode on the ear-side wearing apparatus, obtaining a first potential difference signal corresponding to the bioelectrical signal collected by the left-ear-side signal measurement unit and the reference electrode and a second potential difference signal corresponding to the bioelectrical signal collected by the right-ear-side signal measurement unit and the reference electrode, and then obtaining a difference signal between the first potential difference signal and the second potential difference signal.

When there are a plurality of ear-side signal measurement units and measurement may be performed for a plurality of times, when it is learnt, through measurement, that an impedance between a left-ear-side signal measurement unit and a right-ear-side signal measurement unit is less than the preset threshold, it is determined that measurement can be normally performed on both ear canals corresponding to the two measured measurement units. A potential difference signal corresponding to bioelectrical signals collected by the left-ear-side signal measurement unit and the right-ear-side signal measurement unit that are determined, after being measured, as measurement units that can normally perform measurement is used as the user bioelectrical signal.

When there are a plurality of ear-side signal measurement units and measurement is performed once, when it is learnt, through measurement, that an impedance between a left-ear-side signal measurement unit and a right-ear-side signal measurement unit is less than the preset threshold, it is determined that measurement can be normally performed on both ear canals corresponding to the two measured measurement units. A potential difference signal corresponding to bioelectrical signals collected by the left-ear-side signal measurement unit and the right-ear-side signal measurement unit that are determined, after being measured, as measurement units that can normally perform measurement is used as the user bioelectrical signal; or if it may be considered, based on a measurement result, that the ear-side wearing apparatus can normally perform measurement, any left-ear-side signal measurement unit and any right-ear-side signal measurement unit, or a pre-specified left-ear-side signal measurement unit and a pre-specified right-ear-side signal measurement unit are selected to obtain a potential difference signal corresponding to bioelectrical signals collected by the two measurement units is used as the user bioelectrical signal.

Further, when it is determined that the ear-side wearing apparatus cannot normally perform measurement, the signal collection and attention detection steps may not be performed.

Optionally, when it is determined that the ear-side wearing apparatus is not normally worn, whether a left side or a right side of the ear-side wearing apparatus is normally worn may further be determined. Therefore, optionally, step S50103 may be performed.

S203. When a determining result is that the ear-side wearing apparatus cannot normally perform measurement, determine whether an impedance between two of the plurality of left-ear-side signal measurement units is less than a preset threshold and whether an impedance between two of the plurality of right-ear-side signal measurement units is less than a preset threshold, and use a potential difference signal corresponding to bioelectrical signals collected by two bioelectrical measurement apparatuses that are on one ear canal side and between which an impedance is less than the preset threshold as the user bioelectrical signal.

In this step, a plurality of left-ear-side signal measurement units and a plurality of right-ear-side signal measurement units are required.

When there are a plurality of ear-side signal measurement units and measurement may be performed for a plurality of times, when impedances that are between a left-ear-side signal measurement unit and a right-ear-side signal measurement unit and that are obtained after measurement is performed for a plurality of times are all greater than the preset threshold, it is determined that measurement cannot be normally performed on at least one of ear canals corresponding to the two measured measurement units.

When there are a plurality of ear-side signal measurement units and measurement is performed once, when a measured impedance between a left-ear-side signal measurement unit and a right-ear-side signal measurement unit is greater than the preset threshold, it is determined that measurement cannot be normally performed on at least one of ear canals corresponding to the two measured measurement units. To be specific, when an impedance between one of the left-ear-side signal measurement units and one of the right-ear-side signal measurement units is greater than the preset threshold, it is determined that measurement cannot be normally performed on at least one of ear canals corresponding to the two measured measurement units.

After it is determined that measurement cannot be normally performed on at least one of ear canals corresponding to two measured measurement units, whether an impedance between two of the plurality of left-ear-side signal measurement units is less than the preset threshold and whether an impedance between two of the plurality of right-ear-side signal measurement units is less than the preset threshold are determined. There may also be a plurality of measurement policies. For example, two left/right-ear-side signal measurement units are arbitrarily selected to obtain an impedance value between the two measurement units; or an impedance value between two left/right-ear-side signal measurement units at preset positions is obtained only once, and whether the ear-side wearing apparatus is normally worn on left and right ears is determined based on the obtained impedance value. Alternatively, a priority sequence may be set to perform measurement between two left/right-ear-side signal measurement units, and when the preset threshold is not satisfied, measurement and determining are terminated after measurement is performed for a preset quantity of times. Alternatively, measurement is performed on measurement unit pairs one by one until it is learnt, through measurement, that an impedance between one pair of measurement units is less than the preset value. In this case, it indicates that the ear-side wearing apparatus can normally perform measurement; or otherwise, when it is learnt, through measurement after all measurement operations are completed, that an impedance between any pair of measurement units is not less than the preset value, it indicates that the ear-side wearing apparatus cannot normally perform measurement. Similarly, a specific measurement method performed in a case in which there are a plurality of measurement units on a single side is not limited in this application.

The using a potential difference signal corresponding to bioelectrical signals collected by two bioelectrical measurement apparatuses that are on one ear canal side and between which an impedance is less than the preset threshold as the user bioelectrical signal may be:

using the potential difference signal corresponding to the bioelectrical signals collected by the left-ear-side signal measurement unit and the right-ear-side signal measurement unit as the user bioelectrical signal, where the two signal measurement unit are determined as measurement units that can normally perform measurement, after being measured.

A specific manner of using the potential difference signal corresponding to the bioelectrical signals collected by the left-ear-side signal measurement unit and the right-ear-side signal measurement unit that are determined, after being measured, as measurement units that can normally perform measurement, as the user bioelectrical signal may include: directly using a potential difference signal corresponding to bioelectrical signals collected by two ear-side signal measurement units that are on one side and that can normally perform measurement as the user bioelectrical signal; or configuring a reference electrode on the ear-side wearing apparatus, obtaining a third potential difference signal corresponding to a bioelectrical signal collected by one ear-side signal measurement unit and the reference electrode and a fourth potential difference signal corresponding to a bioelectrical signal collected by the other ear-side signal measurement unit and the reference electrode, and then obtaining a difference signal between the third potential difference signal and the fourth potential difference signal.

There may be a plurality of manners of selecting an ear-side signal measurement unit. For example, two measurement units whose impedance values are to be determined are directly selected to obtain a potential difference signal, or measurement units may be selected according to a preset setting, or a measurement unit may be arbitrarily selected.

In this application, a manner of obtaining a potential difference signal may specifically be implemented by using a software instruction, or may be implemented by using a hardware circuit.

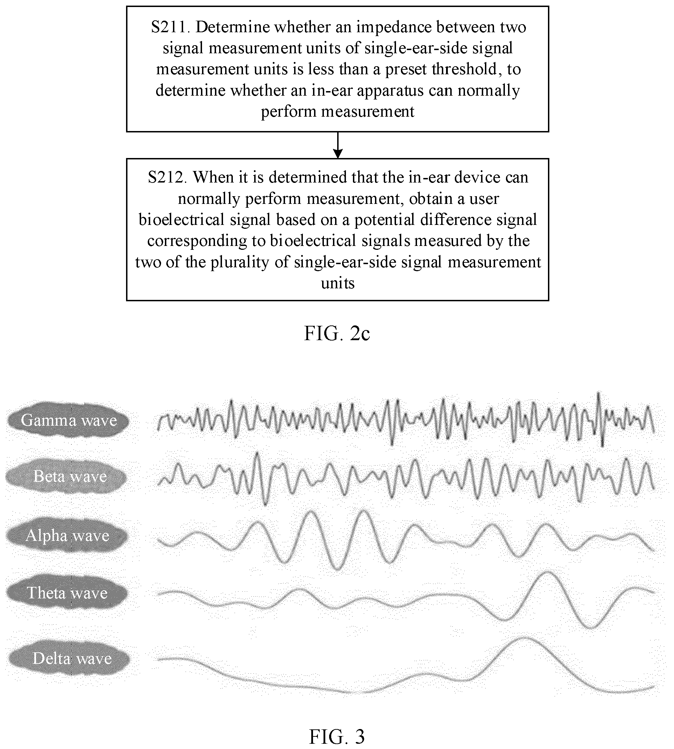

Corresponding to a case in which the ear-side wearing apparatus is a single-side measurement apparatus, that is, the ear-side wearing apparatus includes only a left-ear-side signal measurement unit or a right-ear-side signal measurement unit. In this implementation, a plurality of left-ear-side signal measurement units or a plurality of right-ear-side signal measurement units are required, that is, there are a plurality of single-ear-side signal measurement units. Alternatively, a manner of collecting a bioelectrical signal from the ear side may be shown in FIG. 2c, and further includes the following steps.

S211. Determine whether an impedance between two signal measurement units of the single-ear-side signal measurement units is less than a preset threshold, to determine whether the ear-side wearing apparatus can normally perform measurement.

Whether the impedance between the two signal measurement units of the single-ear-side signal measurement units is less than the preset threshold is determined to determine whether the ear-side wearing apparatus can normally perform measurement (that is, can be normally worn).

Specifically, there may be a plurality of measurement policies. For example, two single-ear-side signal measurement units are arbitrarily selected to obtain an impedance value between the two measurement units; or an impedance value between two single-ear-side signal measurement units at preset positions may be obtained only once, and whether the ear-side wearing apparatus is normally worn on a single ear canal side is determined based on the obtained impedance value. To be specific, whether an impedance between two of the single-ear-side signal measurement units is less than the preset threshold is determined; and if the impedance is less than the threshold, it is determined that the ear-side wearing apparatus is normally worn.

Alternatively, a priority sequence may be set to perform measurement between two single-ear-side signal measurement units, and when the preset threshold is not satisfied, measurement and determining are terminated after measurement is performed for a preset quantity of times. Alternatively, measurement is performed on measurement unit pairs one by one until it is learnt, through measurement, that an impedance between one pair of measurement units is less than the preset value. In this case, it indicates that the ear-side wearing apparatus can normally perform measurement; or otherwise, when it is learnt, through measurement after all measurement operations are completed, that an impedance between any pair of measurement units is not less than the preset value, it indicates that the ear-side wearing apparatus cannot normally perform measurement. Similarly, a specific measurement method performed in a case in which there are a plurality of measurement units on a single side is not limited in this application.

S212. When it is determined that the ear-side wearing apparatus can normally perform measurement, use a potential difference signal corresponding to bioelectrical signals collected by two of the plurality of single-ear-side signal measurement units as the user bioelectrical signal.

When there are a plurality of single-ear-side measurement units and measurement may be performed for a plurality of times, when it is learnt, through measurement, that an impedance between two single-ear-side signal measurement units is less than the preset threshold, it is determined that the ear-side wearing apparatus can normally perform measurement. A potential difference signal corresponding to bioelectrical signals collected by the two single-ear-side signal measurement units that are determined, after being measured, as measurement units that can normally perform measurement is used as the user bioelectrical signal.

A specific manner of using the potential difference signal corresponding to the bioelectrical signals collected by the two single-ear-side signal measurement units that are determined, after being measured, as measurement units that can normally perform measurement, as the user bioelectrical signal may include: directly using a potential difference signal corresponding to bioelectrical signals collected by two ear-side signal measurement units that can normally perform measurement as the user bioelectrical signal; or configuring a reference electrode on the ear-side wearing apparatus, obtaining a fifth potential difference signal corresponding to a bioelectrical signal collected by one ear-side signal measurement unit and the reference electrode and a sixth potential difference signal corresponding to a bioelectrical signal collected by the other ear-side signal measurement unit and the reference electrode, and then obtaining a difference signal between the fifth potential difference signal and the sixth potential difference signal.

When there are a plurality of single-ear-side signal measurement units and measurement is performed once, when it is learnt, through measurement, that an impedance between single-ear-side signal measurement units is less than the preset threshold, it is determined that measurement can be normally performed on both ear canals corresponding to the two measured measurement units. A potential difference signal corresponding to bioelectrical signals collected by the two single-ear-side signal measurement units that are determined, after being measured, as measurement units that can normally perform measurement is used as the user bioelectrical signal; or if it may be considered, based on a measurement result, that the ear-side wearing apparatus can normally perform measurement, any two single-ear-side measurement units or two pre-specified single-ear-side measurement units are selected to obtain a potential difference signal corresponding to bioelectrical signals collected by the two measurement units is used as the user bioelectrical signal.

Potential difference processing mentioned in the foregoing implementation may specifically be performing differential processing on collected bioelectrical signals. Because it is relatively difficult to obtain an electroencephalogram signal from the ear side, especially from an ear canal, strength of the obtained electroencephalogram signal is relatively low, and subsequent signal determining may be greatly affected by noise interference. Therefore, to ensure implementability of obtaining the electroencephalogram signal from the ear side and accuracy of a conclusion obtained through subsequent user attention analysis, targeted denoising processing needs to be performed on a bioelectrical signal collected from the ear canal, and a differential circuit can remove noise in the collected bioelectrical signal. The ear-side wearing apparatus is an electronic product. In a running process, although electromagnetic shielding design has been performed on a circuit, the circuit may be affected by an electrical signal on a circuit board and an electromagnetic wave in air in a special scenario, leading to waveform distortion. In view of this, a differential technology is used, electrodes are attached to both ears, and signals are collected from both ear canals, thereby ensuring signal accuracy.

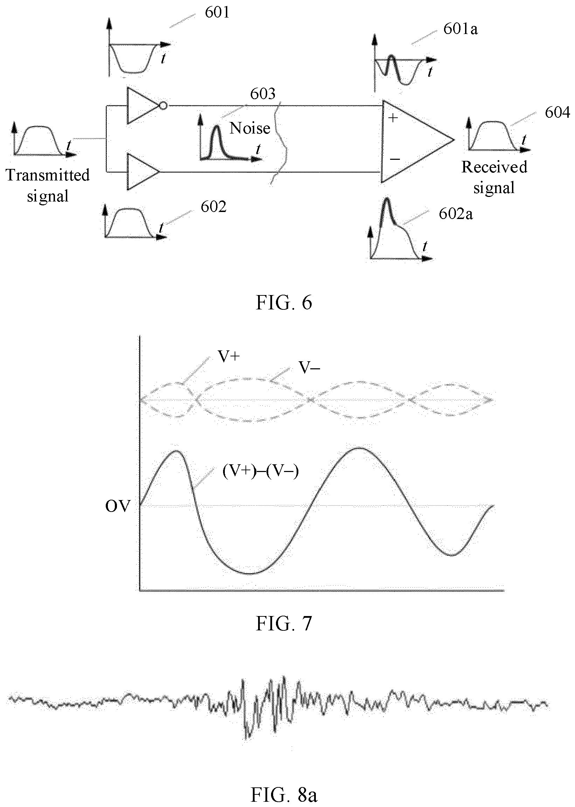

FIG. 6 is a specific principle diagram, and shows a signal receiving circuit model on two ear canals. When external noise exists on a line, interference is eliminated by using a differential circuit. This facilitates subsequent extraction of a correct waveform. 601 represents a left-ear-canal bioelectrical signal, 602 represents a right-ear-canal bioelectrical signal, 603 represents a noise signal, 601a represents a left-ear-canal bioelectrical signal obtained after noise is mixed, 602a represents a right-ear-canal bioelectrical signal obtained after noise is mixed, and 604 represents a bioelectrical signal obtained after differential processing, that is, a first bioelectrical signal. FIG. 6 is merely an example of a case. Alternatively, 601 may represent a right-ear-canal bioelectrical signal, and 602 may represent a left-ear-canal bioelectrical signal.

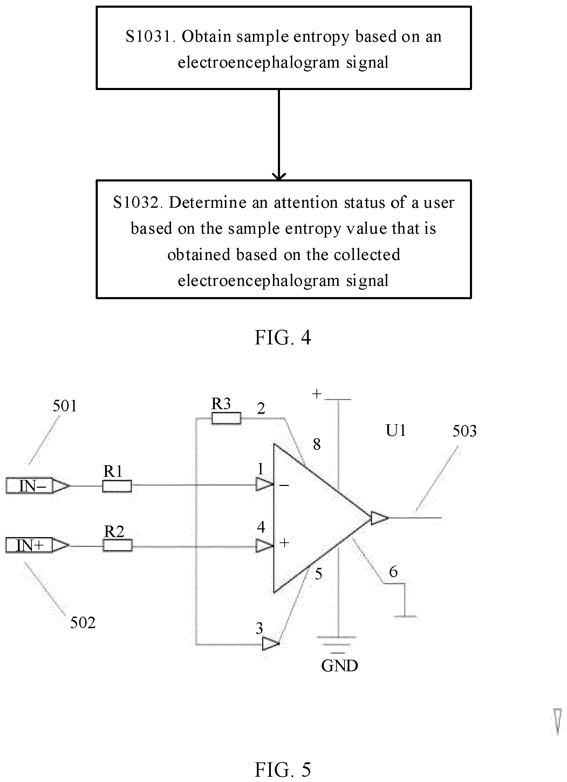

During current design of a differential circuit, usually, the differential circuit is implemented directly by using a chip. A circuit implemented in this embodiment of the present disclosure is shown in FIG. 5. 501 and 502 represent inputs of bioelectrical signals collected from left and right ear canals, and 503 represents a first bioelectrical signal output after performing differential processing on the bioelectrical signals by using the differential circuit. FIG. 7 is a schematic waveform diagram, in which V+ represents a left-ear-canal bioelectrical signal, V- represents a right-ear-canal bioelectrical signal, and (V+)-(V-) represents a bioelectrical signal obtained after differential processing. Similarly, FIG. 7 is merely an example of a case. Alternatively, V+ may represent a right-ear-canal bioelectrical signal, and V- may represent a left-ear-canal bioelectrical signal.

Based on a specific application requirement, an electroencephalogram signal may be extracted from the user bioelectrical signal obtained in S101 in FIG. 2a.

S102. Obtain the user electroencephalogram signal from the user bioelectrical signal.

The bioelectrical signal includes one or more of various characteristic signals of a human body, such as an electrocardiogram ECG signal, an electro-oculogram EOG signal, an electromyogram EMG signal, and an electroencephalogram EEG signal. There may be a plurality of methods for extracting different types of biological characteristic signals through characteristic decomposition. Different types of signals can be extracted based on different spectra of the signals. A more common manner is performing independent component analysis (ICA) by using a blind signal source separation algorithm, to obtain components of a plurality of biological characteristic signals through decomposition. The electroencephalogram signal is extracted in this manner.

In addition to extraction, some conventional processing may be selectively performed on the electroencephalogram signal. The processing may include one or more of conventional bioelectrical signal processing operations such as artifact removal, wavelet analysis, and digital coding, and is used for obtaining a more accurate and real electroencephalogram signal that can reflect a user electroencephalogram characteristic. Alternatively, the processing may be other denoising processing and digital conversion. This is not limited herein. Processing manners and functions of various processing operations are as follows.

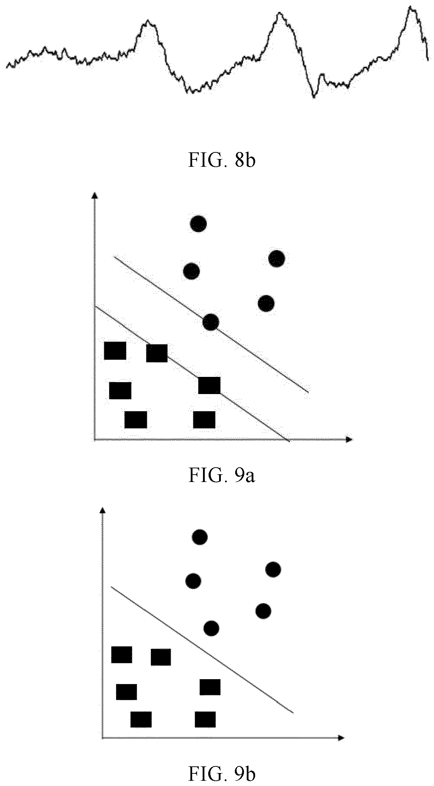

Artifact removal: Electrical phenomena occur at many positions in a human body, a most common phenomenon is nerve conduction. One neuron transfers bioelectricity to a next neuron after receiving a stimulus. Such electrical phenomenon occurs all the time with the survival of human beings. Every tiny expression of a human being is closely correlated to nerve current conduction. In addition to nerve cells, an organ in a human body can also generate bioelectrical signals of different strength. However, during measurement of an electroencephalogram signal, other bioelectrical signals are mixed in the electroencephalogram signal. Because the original electroencephalogram signal cannot be completely extracted, and is basically mixed with different bioelectrical signals from a human body, the electroencephalogram signal is affected greatly or slightly. In addition, an expression and body actions of a human being such as heart beating, a muscle action, a winking action, deep breathing, and skin sweating can also greatly affect an electroencephalogram signal. Moreover, a temperature difference also causes different changes in strength of a bioelectrical signal. If environment temperature is relatively low, a few people shiver and tremble. All these actions have relatively large amplitudes, and can also cause interference to an electroencephalogram signal. FIG. 8a shows a muscle artifact generated by a neck joint action, and FIG. 8b shows an ocular artifact generated by winking. These artifacts are mixed with a useful electroencephalogram signal. As a result, a data processing difficulty is increased. Therefore, after the electroencephalogram signal is collected, the artifacts in the electroencephalogram signal need to be removed. Wavelet analysis: It is a time-frequency analysis method. Because an electroencephalogram signal is an unsteady signal, details cannot be well extracted through conventional Fourier transform (only frequency information can be extracted and time information cannot be extracted). Wavelet transform is a signal analysis method, and can well reflect a time characteristic of a signal in frequency domain. A local characteristic of a signal can be well represented through wavelet analysis.

Digital coding: Digital coding is performed on an electroencephalogram signal to convert the electroencephalogram signal into a digital signal.

Artifact removal processing may be performed on a bioelectrical signal, or may be performed on an electroencephalogram signal obtained after characteristic extraction is performed.

The obtained electroencephalogram signal may be used to perform user attention analysis and determining, and step S103 may further be performed on the extracted user electroencephalogram signal.

S103. Obtain an attention type of the user based on the user electroencephalogram signal and a machine learning model. S103 specifically includes the following.

The attention type of the user is analyzed based on the obtained electroencephalogram signal. A common processing manner is performing attention characteristic extraction on the obtained electroencephalogram signal. The electroencephalogram signal, that is, the electroencephalogram EGG (electroencephalogram) signal, is an external manifestation of a brain activity. Different brain activities are manifested as electroencephalogram signals with different characteristics. Research shows that a status of a person can be clearly detected by using a detected electroencephalogram signal.

In usual human activities, $\alpha$, $\beta$, $\gamma$, $\theta$, and $\delta$ wave bands are generated, and waveforms thereof are shown in FIG. 3.

$\delta$ wave: A frequency of the $\delta$ wave is distributed from 1 Hz to 4 Hz, and a wave amplitude thereof is between 20 uv and 200 uv. The $\delta$ wave is relatively obvious in a parietal lobe and a pituitary, and is relatively obvious in an infant period or an immature period of intellectual development. Like the θ wave, the δ wave is a slow wave. In a normal case, the δ wave exists only in a state in which there is an extreme lack of oxygen, a deep sleep state, a state in which there is a cerebral disease, or the like.

θ wave: A frequency of the θ wave is distributed from 4 Hz to 7 Hz, and a wave amplitude thereof is between 20 uf and 40 uf. The θ wave is a slow wave. The θ wave mainly appears in occipital and parietooccipital regions, and positions that are corresponding to the θ wave and that are in occipital and parietooccipital regions are bilaterally symmetrical. A θ wave can usually be detected when a person is sleepy or is in a light-sleep state. In addition, there is a universal relationship between the θ wave and a psychological state of the person. Usually, when the person feels depressed, frustrated, or sleepy, a central nervous system is in a depressed state, and the wave appears.

α wave: A frequency of the α wave is distributed from 8 Hz to 12 Hz, and a wave amplitude thereof is between 25 uf and 75 uf. The α wave mainly appears in a parietooccipital region, basically keeps synchronized on two sides thereof, and is a basic rhythm that an EEG of a normal person should have. When an individual is thinking or in a rest state, the wave is relatively obvious, and when the individual undertakes a targeted activity, opens eyes, or receives other stimuli, the wave disappears, and a β wave appears instead.

β wave: A frequency of the β wave is distributed from 14 Hz to 30 Hz, and a wave amplitude thereof is approximately half of that of the δ wave. The β wave mainly appears in a forehead region and a central region. The frequency of the wave significantly represents an excitement degree of a cerebral cortex, and the wave appears when an individual is awake or asleep.

Therefore, by analyzing a waveform characteristic of the obtained electroencephalogram signal, a current attention status of the user, that is, whether the user is awake or asleep and whether attention of the user is focused or not, can be determined.

In a specific embodiment of this application, step S103 may further include steps shown in FIG. 4.

S1031. Obtain sample entropy based on the electroencephalogram signal.

An obtaining process of obtaining the sample entropy based on the electroencephalogram signal includes the following steps.

A. Intercept the electroencephalogram signal of a preset time length, and obtain N signal sampling points u(1), u(2), ..., and u(N) from the electroencephalogram signal of the preset time length.

Usually, the sampling points are sampling points at an equal time interval, and the intercepted preset time length is optional.

B. Sequentially intercept m sampling points based on the N signal sampling points by using each of u(1), u(2), ..., and u(N-m+1) as a start point, to construct N-m+1 m-dimensional vectors.

The constructed N-m+1 m-dimensional vectors are X(1), X(2), ..., and X(N-m+1), where X(i)=[u(i), u(i+1), ..., u(i+m-1)], 1≤i≤N-m+1, and m<N.

C. Calculate, for each of the N-m+1 m-dimensional vectors, a ratio of a quantity of vectors that are in all the other vectors and whose distances to the m-dimensional vector are less than r to a quantity of all the other vectors, and calculate an average value of the obtained N-m+1 ratios to obtain a first average value.

For each m-dimensional vector in the N-m+1 vectors, a quantity of vectors that satisfy the following condition is calculated.

$B_i(r)$=(number of X(j) such that d[X(i), X(j)]<r)/(N-m), where i≠j, a value range of i is [1, N-m+1], a value range of j is [1, N-m+1] except i, and r is a preset value. For example, a value of r may be related to a standard deviation $\delta$ of the sampling points, and the value may range from 0.1 $\delta$ to 0.3 $\delta$. d[X(i), X(j)] is defined as d[X(i), X(j)]=max|u(a)-u*(a)|, where i≠j, u(a) is an element of a vector X(i), u*(a) is an element in a corresponding dimension of a vector X(j), and d represents a distance between the vectors X(i) and X(j). The distance between the vectors X(i) and X(j) is determined by a maximum difference in differences between corresponding elements. For example, if X(1)=[2, 3, 4, 6], and X(2)=[4, 5, 7, 10], a maximum difference between corresponding elements is |6-10|=4. Therefore, d[X(1), X(2)]=4. An average value of $B_i(r)$ corresponding to all values of i is calculated and denoted as $B_m(r)$, that is,

$$B_m(r) = (N - m + 1)^{-1} \sum_{i\in[1,N-m+1]} B_i(r)$$

D. Sequentially intercept m+1 sampling points based on the N signal sampling points by using each of u(1), u(2), ..., and u(N-m) as a start point, to construct N-m (m+1)-dimensional vectors.

The constructed N-m (m+1)-dimensional vectors are Y(1), Y(2), ..., and Y(N-m), where X(i)=[u(i), u(i+1), ..., u(i+m)], 1≤i≤N-m, and m<N.

E. Calculate, for each of the N-m (m+1)-dimensional vectors, a ratio of a quantity of vectors that are in all the other vectors and whose distances to the (m+1)-dimensional vector are less than r to a quantity of all the other vectors, and calculate an average value of the obtained N-m ratios to obtain a second average value.

For each (m+1)-dimensional vector in the N-m vectors, a quantity of vectors that satisfy the following condition is calculated.

$A_i(r)$=(number of Y(j) such that d[Y(i), Y(j)]≤r)/(N-m-1), where i≠j, a value range of i is [1, N-m], a value range of j is [1, N-m] except i, and r is a preset value. For example, a value of r may be related to a standard deviation $\delta$ of the sampling points, and the value may range from 0.1 $\delta$ to 0.3 $\delta$. d[Y(i), Y(j)] is defined as d[Y(i), Y(j)]=max|u(a)-u*(a)|, where i≠j, u(a) is an element of a vector Y, and d represents a distance between vectors Y(i) and Y(j) and is determined by a maximum difference between corresponding elements. An average value of $A_i(r)$ corresponding to all values of i is calculated and denoted as $A_m(r)$, that is,

$$A_m(r) = (N - m)^{-1} \sum_{i \in [1, N-m]} A_i(r).$$

F. Calculate a sample entropy (SampEn) value based on a ratio of the first average value to the second average value.

SampEn=lim(N→∞) {-ln[$A_m(r)$/$B_m(r)$]} holds true.

A sequence of A to F is variable. For example, a sequence between implementation of B and C and implementation of D and E is variable. D and E may be performed before B and C, or D and E may be implemented at a same time as B and C, or time for implementing B and C and time for implementing D and E may partially overlap with each other.

S1032. Determine an attention status of the user based on the sample entropy value that is obtained based on the collected electroencephalogram signal.

The attention status of the user is determined based on the obtained sample entropy value. In a specific implementation process, there may be a plurality of implementations. For example, the user or product research and development personnel may set one or more preset values based on historical experience, for example, a segmentation value used to distinguish whether attention is focused or distracted and a segmentation value used to distinguish whether the user is awake or asleep. For example, for the segmentation value used to distinguish whether attention is focused or distracted, when the sample entropy value is greater than or is greater than or equal to the segmentation value, it indicates that the attention is focused; or when the sample entropy value is less than or equal to or is less than the segmentation value, it indicates that the attention is distracted. The segmentation value and a quantity of segmentation values are determined based on a quantity of to-be-distinguished attention statuses and a type of an attention status.

In addition, machine learning model training may be performed by using an SVM classifier to obtain a segmentation value, and the attention type of the user may be determined based on the segmentation value and the sample entropy value.

In a model training manner, a plurality of electroencephalogram signal samples in specific duration that are corresponding to different attention types are used, sample entropy values of the electroencephalogram signal samples are calculated, and SVM model training is performed by using samples constructed by using the sample entropy values and the corresponding attention types. Then, a trained model is used for subsequent attention analysis. To be specific, a sample entropy value of a corresponding electroencephalogram signal is input, and a corresponding attention type or an attention type probability is output.

SVM is a discrimination classifier defined by a classification hyperplane. A group of labeled training samples are provided, and an optimal hyperplane is output by using an algorithm, to perform classification on a new sample (test sample). FIG. 9a and FIG. 9b are schematic diagrams of obtaining an optimal hyperplane. Dots and squares represent two different types of data. For a linear separable set including two-dimensional coordinate points, if a segmentation line that is as far away as possible from both types of sample points can be found, the segmentation line is considered as an optimal hyperplane in two-dimensional coordinate space, that is, a solid line in FIG. 9b. SVM machine learning is to find a hyperplane, and the hyperplane can ensure that a distance between training samples that are nearest to the hyperplane is farthest, while distinguishing between two types of data. In other words, a training sample boundary is maximized by using an optimal segmentation hyperplane.

After a plurality of sample entropy values and attention statuses corresponding to the sample entropy values are input, the SVM classifier outputs one or more segmentation values through SVM machine learning, to determine an attention status corresponding to the sample entropy of the user electroencephalogram signal. There may be one or more segmentation values, for example, a segmentation value used to distinguish whether attention is focused or distracted and a segmentation value used to distinguish whether the user is awake or asleep. For example, for the segmentation value used to distinguish whether attention is focused or distracted, when the sample entropy value is greater than or is greater than or equal to the segmentation value, it indicates that the attention is focused; or when the sample entropy value is less than or equal to or is less than the segmentation value, it indicates that the attention is distracted. In a sample entropy analysis method, only data in a relatively low dimension is required to obtain a robust estimated value. Therefore, the sample entropy analysis method is an attention analysis method with relatively desirable anti-noise and anti-interference effects.

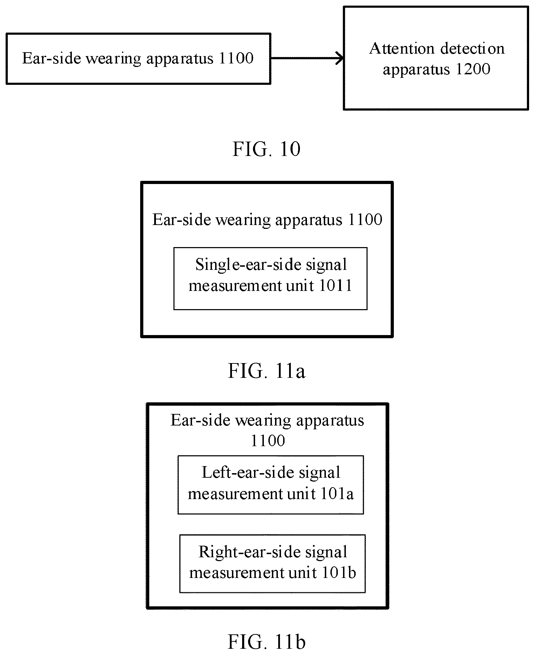

FIG. 10 is a diagram of an example of an attention detection system according to an embodiment of the present invention. The system includes an ear-side wearing apparatus 1100 and an attention detection apparatus 1200.

The ear-side wearing apparatus 11000 is configured to: collect a user bioelectrical signal from an ear side, and obtain an electroencephalogram signal from the user bioelectrical signal.

The ear-side wearing apparatus 11000 may specifically be a single-side measurement apparatus or a dual-side measurement apparatus. A structure of the ear-side wearing apparatus 11000 when the ear-side wearing apparatus 11000 is a single-side measurement apparatus is shown in FIG. 11a. A single-ear-side signal measurement unit 1011 is configured to obtain a user bioelectrical signal from a left ear canal or a right ear canal.

A structure of the ear-side wearing apparatus 11000 when the ear-side wearing apparatus 11000 is a dual-side measurement apparatus is shown in FIG. 11b. A left-ear-side signal measurement unit 101a is configured to obtain a user bioelectrical signal from the left ear canal, and a right-ear-side signal measurement unit 101b is configured to obtain a user bioelectrical signal from the right ear canal.

Corresponding to a case in which the ear-side wearing apparatus 11000 is a dual-side measurement apparatus, that is, the ear-side wearing apparatus includes the left-ear-side signal measurement unit 101a and the right-ear-side signal measurement unit 101b, the ear-side wearing apparatus 11000 determines whether an impedance between the left-ear-side signal measurement unit 101a and the right-ear-side signal measurement unit 101b is less than a preset threshold, to determine whether the ear-side wearing apparatus can normally perform measurement; and when determining that the ear-side wearing apparatus can normally perform measurement, obtains the user bioelectrical signal based on a potential difference signal corresponding to the bioelectrical signal collected by the left-ear-side signal measurement unit and the bioelectrical signal collected by the right-ear-side signal measurement unit; or when a determining result is that the ear-side wearing apparatus cannot normally perform measurement, determines whether an impedance between two of a plurality of left-ear-side signal measurement units is less than the preset threshold and whether an impedance between two of a plurality of right-ear-side signal measurement units is less than the preset threshold, and obtains the user bioelectrical signal based on a potential difference signal corresponding to bioelectrical signals collected by two bioelectrical measurement apparatuses that are on one ear canal side and between which an impedance is less than the preset threshold. For a specific determining manner, refer to steps S201 to S6203.

Corresponding to a case in which the ear-side wearing apparatus 11000 is a single-side measurement apparatus measurement apparatus, that is, the ear-side wearing apparatus 11000 includes only a left-ear-side signal measurement unit 1011 or a right-ear-side signal measurement unit 1011, in this implementation, a plurality of left-ear-side signal measurement units or a plurality of right-ear-side signal measurement units are required, that is, there are a plurality of single-ear-side signal measurement units. The ear-side wearing apparatus 11000 determines whether an impedance between two signal measurement units of the single-ear-side signal measurement unit is less than a preset threshold, to determine whether the ear-side wearing apparatus can normally perform measurement; and when determining that the ear-side wearing apparatus can normally perform measurement, obtains the user bioelectrical signal based on a potential difference signal corresponding to bioelectrical signals collected by the two signal measurement units of the plurality of single-ear-side signal measurement units. For a specific determining manner, refer to steps S211 and S212. The attention detection apparatus 1200 is configured to detect an attention type of a user based on the electroencephalogram signal.

Details of specifically collecting the electroencephalogram signal by the ear-side wearing apparatus 11000 have been described in S101 and S102 in FIG. 2, and technical details of detecting user attention by the attention detection apparatus 1200 have also been described in S102 in FIG. 2 and FIG. 4. Details are not described herein again.

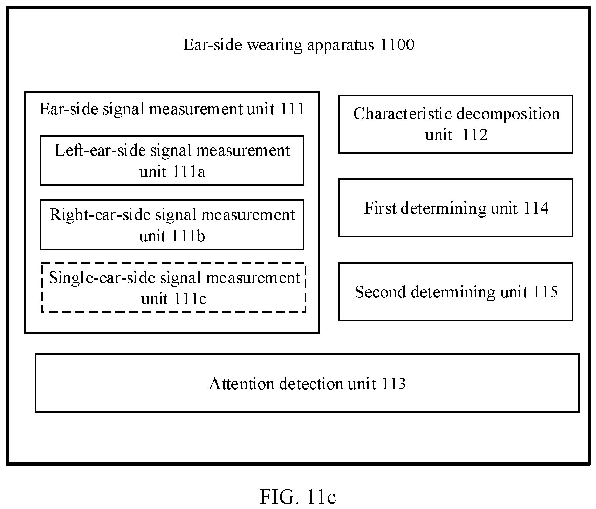

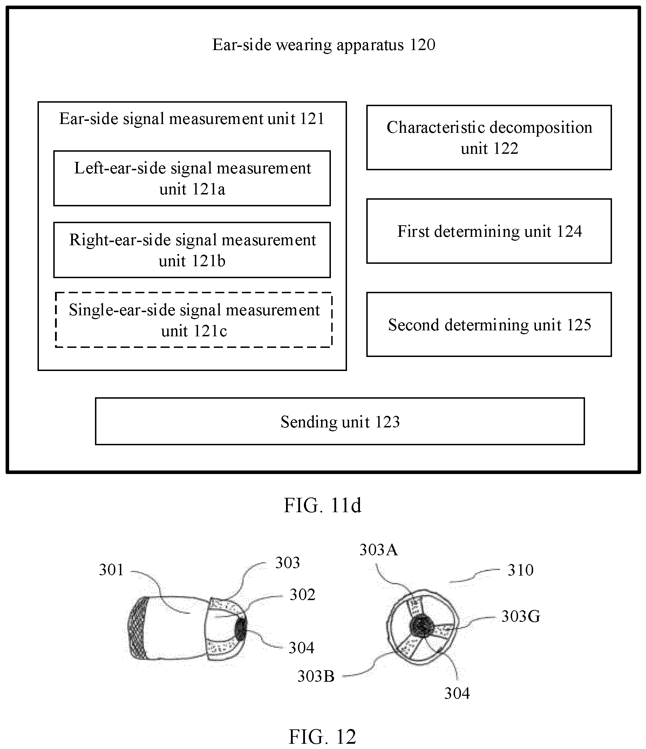

FIG. 11c is a structural diagram of an example of an ear-side wearing apparatus 11000 having an attention detection capability according to an embodiment of the present disclosure. In some embodiments of the present invention, an attention detection apparatus and the ear-side wearing apparatus may be integrated together. FIG. 11 correspondingly shows an ear-side wearing apparatus 1100 integrated with an attention detection function according to this embodiment of this application. The apparatus includes an ear-side signal measurement unit 111, a characteristic decomposition unit 112, an attention detection unit 113, and a first determining unit 114.

The ear-side signal measurement unit 111 is configured to collect a user bioelectrical signal from an ear side. Optionally, the ear-side signal measurement unit 111 may include a left-ear-side signal measurement unit 111a and a right-ear-side signal measurement unit 111b. When the ear-side wearing apparatus 1100 is a single-side measurement apparatus, the ear-side signal measurement unit 111 may include only a single-ear-side signal measurement unit 111c.

The characteristic decomposition unit 112 is configured to obtain an electroencephalogram signal from the user bioelectrical signal.

The attention detection unit 113 is configured to obtain an attention classification result of a user based on the electroencephalogram signal and a machine learning model. For a specific analysis manner, refer to the foregoing specific embodiment. Details are not described herein again.

The first determining unit 114 is configured to: determine whether an impedance between two of ear-side signal measurement units is less than a preset threshold; and when the impedance between the two ear-side signal measurement units is less than the preset threshold, use a potential difference signal corresponding to bioelectrical signals collected and measured by the two ear-side signal measurement units as the user bioelectrical signal.

Corresponding to a case of dual-side measurement, the ear-side wearing apparatus may optionally include a second determining unit.

The first determining unit 114 is configured to: determine whether an impedance between the left-ear-side signal measurement unit and the right-ear-side signal measurement unit is less than the preset threshold (a specific determining manner has been described above, and is not described herein again); and when the impedance between the left-ear-side signal measurement unit and the right-ear-side signal measurement unit is less than the preset threshold, use a potential difference signal corresponding to a bioelectrical signal collected by the left-ear-side signal measurement unit and a bioelectrical signal collected by the right-ear-side signal measurement unit as the user bioelectrical signal.

The second determining unit 115 is configured to: when the first determining unit 114 determines that the ear-side wearing apparatus cannot normally perform measurement (a specific determining manner has been described above, and is not described herein again), determine whether an impedance between two of a plurality of left-ear-side signal measurement units is less than the preset threshold and whether an impedance between two of a plurality of right-ear-side signal measurement units is less than the preset threshold; and use a potential difference signal corresponding to bioelectrical signals collected by two bioelectrical measurement apparatuses that are on one ear canal side and between which an impedance is less than the preset threshold as the user bioelectrical signal. The second determining unit 115 is an optional unit, and the second determining unit 115 is applied to a case in which there are a plurality of left-ear-side signal measurement units and a plurality of right-ear-side signal measurement units.

Corresponding to a case of single-side measurement, the first determining unit 114 included in the ear-side wearing apparatus is configured to determine whether an impedance between two of single-ear-side signal measurement units is less than the preset threshold (a specific determining manner has been described above, and is not described herein again); and when the impedance between the two single-ear-side signal measurement units is less than the preset threshold, use a potential difference signal corresponding to bioelectrical signals collected by the two of the plurality of single-ear-side measurement units as the user bioelectrical signal.

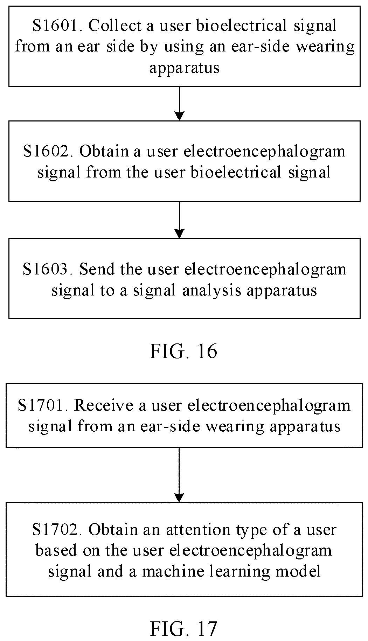

An embodiment of the present disclosure further discloses a method for measuring a user electroencephalogram signal. As shown in FIG. 16, steps S1601 and S1602 are the same as those in FIG. 2, and S1603 is sending the electroencephalogram signal to a signal analysis apparatus. The signal analysis apparatus in this embodiment of this application may specifically be an attention detection apparatus.

Correspondingly, an embodiment of the present disclosure further discloses an ear-side wearing apparatus for measuring a user-related signal. As shown in FIG. 11d, an ear-side signal measurement unit 121 is configured to collect a user bioelectrical signal from an ear side. Optionally, the ear-side signal measurement unit 121 may include a left-ear-side signal measurement unit 121a and a right-ear-side signal measurement unit 121b.

When the ear-side wearing apparatus 120 is a single-side measurement apparatus, the ear-side signal measurement unit 121 may include only a single-ear-side signal measurement unit 121c.

A characteristic decomposition unit 122 is configured to obtain an electroencephalogram signal from the user bioelectrical signal.

A sending unit 123 is configured to send the biological characteristic signal to a signal analysis apparatus. The signal analysis apparatus in this embodiment of this application may specifically be an attention detection apparatus.

A first determining unit 124 is configured to: determine whether an impedance between two of ear-side signal measurement units is less than a preset threshold; and when the impedance between the two ear-side signal measurement units is less than the preset threshold, use a potential difference signal corresponding to bioelectrical signals collected and measured by the two ear-side signal measurement units as the user bioelectrical signal.

Corresponding to a case of dual-side measurement, the ear-side wearing apparatus may optionally include a second determining unit 125.

The first determining unit 124 is configured to: determine whether an impedance between the left-ear-side signal measurement unit and the right-ear-side signal measurement unit is less than the preset threshold (a specific determining manner has been described above, and is not described herein again); and when the impedance between the left-ear-side signal measurement unit and the right-ear-side signal measurement unit is less than the preset threshold, use a potential difference signal corresponding to a bioelectrical signal collected by the left-ear-side signal measurement unit and a bioelectrical signal collected by the right-ear-side signal measurement unit as the user bioelectrical signal.

The second determining unit 125 is configured to: when the first determining unit 124 determines that the ear-side wearing apparatus cannot normally perform measurement (a specific determining manner has been described above, and is not described herein again), determine whether an impedance between two of a plurality of left-ear-side signal measurement units is less than the preset threshold and whether an impedance between two of a plurality of right-ear-side signal measurement units is less than the preset threshold, and use a potential difference signal corresponding to bioelectrical signals collected by two bioelectrical measurement apparatuses that are on one ear canal side and between which an impedance is less than the preset threshold as the user bioelectrical signal. The second determining unit 125 is an optional unit, and the second determining unit 125 is applied to a case in which there are a plurality of left-ear-side signal measurement units and a plurality of right-ear-side signal measurement units.

Corresponding to a case of single-side measurement, the first determining unit 124 in the ear-side wearing apparatus is configured to determine whether an impedance between two of single-ear-side signal measurement units is less than the preset threshold (a specific determining manner has been described above, and is not described herein again); and when the impedance between the two single-ear-side signal measurement units is less than the preset threshold, use a potential difference signal corresponding to bioelectrical signals collected by the two of the plurality of single-ear-side measurement units as the user bioelectrical signal.

FIG. 12 is a schematic structural diagram of a specific product of the ear-side wearing apparatus in FIG. 11. The ear-side wearing apparatus may be in a plurality of forms, for example, may be in an earphone form or may be in an earplug form. The ear-side wearing apparatus provided in this example is in an earplug form. However, no limitation is set thereto in this application. The ear-side wearing apparatus includes an earplug body 301, a flexible electrode carrier 302, and a plurality of surface flexible electrodes 303. The flexible electrode carrier 302 provides sufficient elastic support to ensure that the plurality of flexible electrodes 303 attached to a surface of the flexible electrode carrier 302 is closely attached to a surface of an ear side of a user, thereby ensuring that an electroencephalogram signal of the user is stably collected. A part 310 illustrates an example of a composition of the surface flexible electrode 303, including a biosensing flexible electrode 303A, a biosensing flexible electrode 303B, and a grounded common flexible electrode 303 G that are distributed at equal angles of 120 degree, and 304 represents an earplug hole. In some other feasible embodiments, there may be only one or two biosensing flexible electrodes 303 attached to the surface of the flexible electrode carrier 302, while the earplug body 301 is connected to the grounded common flexible electrode. Alternatively, in some other feasible embodiments, the grounded common flexible electrode may be implemented in a plurality of manners such as an electrode contact on an auricle scaffold. FIG. 13 is a schematic diagram of wearing the ear-side wearing apparatus in an earplug form in FIG. 12. 401 represents an ear canal of a user, 402 represents an in-ear earplug for electroencephalogram signal measurement, 403 represents a flexible electrode, and 404 represents an auricle of the user. It can be learned from FIG. 3 that, during wearing of the ear-side wearing apparatus, a plurality of flexible electrodes 403 on a surface of a flexible electrode carrier are closely attached to an inner surface of the ear canal 401 of the user, and forms a measurement system with a head of the user. Although not shown in the figure, the ear-side wearing apparatus may further include a communications module configured to receive or send an electroencephalogram signal, and may optionally include an attention detection unit configured to analyze an attention type of the user by using the electroencephalogram signal.

The ear-side signal measurement units in FIG. 11a to FIG. 11d may be implemented by using flexible electrodes.

An embodiment of the present disclosure further discloses a method for analyzing a user-related signal. As shown in FIG. 17, step S1702 is the same as S603 in FIG. 7, and S1701 is receiving an electroencephalogram signal from an ear-side wearing apparatus.

Correspondingly, an embodiment of the present disclosure further discloses an attention detection apparatus 130, as shown in FIG. 14. The apparatus includes:

a receiving unit 131, configured to receive an electroencephalogram signal from an ear-side wearing apparatus; and
an attention detection unit 132, configured to obtain an attention type of a user based on the electroencephalogram signal.

Correspondingly, the attention detection unit 132 analyzes the attention type of the user based on the electroencephalogram signal. The attention detection unit may be a terminal device of the user such as a mobile phone, or another wearable or portable terminal, or may be a server disposed on a cloud side.

The attention detection unit includes a sample entropy obtaining module and an attention recognition module.

The sample entropy obtaining module is configured to obtain sample entropy based on the electroencephalogram signal.

An obtaining process of obtaining the sample entropy based on the electroencephalogram signal includes the following steps.

A. Intercept the electroencephalogram signal of a preset time length, and obtain N signal sampling points u(1), u(2), ..., and u(N) from the electroencephalogram signal of the preset time length.

Usually, the sampling points are sampling points at an equal time interval, and the intercepted preset time length of the electroencephalogram signal may be set depending on an analysis requirement.

B. Sequentially intercept m sampling points based on the N signal sampling points by using each of u(1), u(2), ..., and u(N-m+1) as a start point, to construct N-m+1 m-dimensional vectors.

The constructed N-m+1 m-dimensional vectors are X(1), X(2), ..., and X(N-m+1), where X(i)=[u(i), u(i+1), ..., u(i+m-1)], $1 \leq i \leq N-m+1$, and m<N.

C. Calculate, for each of the N-m+1 m-dimensional vectors, a ratio of a quantity of vectors that are in all the other vectors and whose distances to the m-dimensional vector are less than r to a quantity of all the other vectors, and calculate an average value of the obtained N-m+1 ratios to obtain a first average value.

For each m-dimensional vector in the N-m+1 vectors, a quantity of vectors that satisfy the following condition is calculated.

$B_i(r)$=(number of X(j) such that d[X(i), X(j)]$\leq$r)/(N-m), where i$\neq$j, a value range of i is [1, N-m+1], a value range of j is [1, N-m+1] except i, and r is a preset value. For example, a value of r may be related to a standard deviation $\delta$ of the sampling points, and the value may range from 0.1 $\delta$ to 0.3 $\delta$. d[X(i), X(j)] is defined as d[X(i), X(j)]=max|u(a)-u*(a)|, where i≠j, u(a) is an element of a vector X(i), u*(a) is an element in a corresponding dimension of a vector X(j), and d represents a distance between the vectors X(i) and X(j). The distance between the vectors X(i) and X(j) is determined by a maximum difference in differences between corresponding elements. For example, if X(1)=[2, 3, 4, 6], and X(2)=[4, 5, 7, 10], a maximum difference between corresponding elements is 16-101=4. Therefore, d[X(1), X(2)]=4. An average value of $B_i(r)$ corresponding to all values of i is calculated and denoted as $B_m(r)$, that is,

$$B_m(r) = (N - m + 1)^{-1} \sum_{i \in [1, N-m+1]} B_i(r)$$

.

D. Sequentially intercept m+1 sampling points based on the N signal sampling points by using each of u(1), u(2), ..., and u(N-m) as a start point, to construct N-m (m+1)-dimensional vectors.

The constructed N-m (m+1)-dimensional vectors are Y(1), Y(2), ..., and Y(N-m), where X(i)=[u(i), u(i+1), ..., u(i+m)], 1≤i≤N-m, and m<N.

E. Calculate, for each of the N-m (m+1)-dimensional vectors, a ratio of a quantity of vectors that are in all the other vectors and whose distances to the (m+1)-dimensional vector are less than r to a quantity of all the other vectors, and calculate an average value of the obtained N-m ratios to obtain a second average value.

For each (m+1)-dimensional vector in the N-m vectors, a quantity of vectors that satisfy the following condition is calculated.

$A_i(r)$=(number of Y(j) such that d[Y(i), Y(j)]≤r)/(N-m-1), where i≠j, a value range of i is [1, N-m], a value range of j is [1, N-m] except i, and r is a preset value. For example, a value of r may be related to a standard deviation $\delta$ of the sampling points, and the value may range from 0.1 $\delta$ to 0.3 $\delta$. d[Y(i), Y(j)] is defined as d[Y(i), Y(j)]=max|u(a)-u*(a)|, where i≠j, u(a) is an element of a vector Y, and d represents a distance between vectors Y(i) and Y(j) and is determined by a maximum difference between corresponding elements. An average value of $A_i(r)$ corresponding to all values of i is calculated and denoted as $A_m(r)$, that is,

$$A_m(r) = (N - m)^{-1} \sum_{i \in [1, N-m]} A_i(r)$$

.

F. Calculate a sample entropy (SampEn) value based on a ratio of the first average value to the second average value.
SampEn=lim(N→∞) {-ln[$A_m(r)/B_m(r)$] } holds true.

A sequence of A to F is variable. For example, a sequence between implementation of B and C and implementation of D and E is variable. D and E may be performed before B and C, or D and E may be implemented at a same time as B and C, or time for implementing B and C and time for implementing D and E may partially overlap with each other.

The attention recognition module is configured to determine an attention status of the user based on the sample entropy value that is obtained based on the collected electroencephalogram signal.

The attention recognition module may include an SVM classifier and a determining module.

The SV classifier is configured to perform machine learning to obtain a segmentation value. Specifically, after a plurality of sample entropy values and attention statuses corresponding to the sample entropy values are input, the SVM classifier may output one or more segmentation values through SVM machine learning, to determine an attention status corresponding to the sample entropy of the user electroencephalogram signal.

The SVM classifier may be disposed in the attention recognition module or may be disposed in another apparatus to perform training to obtain a segmentation value, and then send the segmentation value to the attention recognition module. Alternatively, a segmentation value is manually set by the user or a developer based on a training result.

The determining module is configured to determine the attention type of the user based on the segmentation value and the sample entropy value.

There may be one or more segmentation values, for example, a segmentation value used to distinguish whether attention is focused or distracted and a segmentation value used to distinguish whether the user is awake or asleep. For example, for the segmentation value used to distinguish whether attention is focused or distracted, when the sample entropy value is greater than or is greater than or equal to the segmentation value, it indicates that the attention is focused; or when the sample entropy value is less than or equal to or is less than the segmentation value, it indicates that the attention is distracted.

For specific technical implementation details, refer to related descriptions in FIG. 2.

A specific implementation form of the attention detection apparatus 130 may be a handheld terminal, a vehicle-mounted terminal, or another apparatus that can be used for performing calculation and analysis on an electroencephalogram signal.

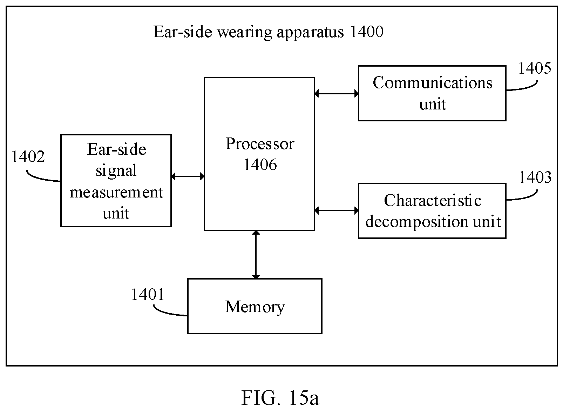

FIG. 15a is correspondingly a schematic structural diagram of a processor of an ear-side wearing apparatus according to an embodiment of this application.

As shown in FIG. 15a, the ear-side wearing apparatus 1400 integrated with an attention detection function may include one or more processors 1406, one or more memories 1401, and a characteristic decomposition unit 1403. In specific implementation, the ear-side wearing apparatus may further include a communications unit 1405. The processor 1406 may be connected to all components such as the memory 1401, a measurement electrode 1402, and the characteristic decomposition circuit 1403 by using a bus. The components are separately described as follows.

The processor 1406 is a control center of the ear-side wearing apparatus, and is connected to the components of the ear-side wearing apparatus by using various interfaces and lines. In a possible embodiment, the processor 1406 may further include one or more processing cores. The processor 1400 may determine, by executing program instructions, whether the measurement electrode can normally perform measurement (whether the ear-side wearing apparatus can normally perform measurement), and perform user attention analysis based on a measurement signal. The processor 1406 may be a dedicated processor or may be a general-purpose processor. When the processor 1406 is a general-purpose processor, the processor 1406 runs or executes software programs (instructions) and/or a module that are/is stored in the memory 1401.

The memory 1401 may include a high-speed random access memory, and may further include a nonvolatile memory, for example, at least one magnetic disk storage device, a flash memory device, or another volatile solid-state storage device. Correspondingly, the memory 1401 may further include a memory controller, to enable the processor 1400 and an input unit to access the memory 1401. The memory 1401 may be specifically configured to store the software programs (instructions) and a collected user bioelectrical signal.

The ear-side signal measurement unit 1402 is configured to collect a user bioelectrical signal from an ear side. Optionally, the ear-side signal measurement unit 1402 may include a left-ear-side signal measurement unit and a right-ear-side signal measurement unit. When the ear-side wearing apparatus 1400 is a single-side measurement apparatus, the ear-side signal measurement unit 1402 may include only a single-ear-side signal measurement unit. The ear-side signal measurement unit 1402 is usually implemented by hardware. For example, the ear-side signal measurement unit 1402 may be an electrode. There may be one or more ear-side signal measurement units 1402.

The characteristic decomposition unit 1403 is configured to obtain an electroencephalogram signal from the user bioelectrical signal. The characteristic decomposition unit 1403 is usually implemented by hardware, for example, a characteristic decomposition circuit or an ICA component.

The communications unit 1405 is configured to establish a communication connection to the ear-side wearing apparatus and another device by using a wireless or wired communications technology such as a cellular mobile communications technology, a WLAN technology, or a Bluetooth technology.

A person skilled in the art can understand that the ear-side wearing apparatus in this embodiment of this application may include more or fewer components than those shown in the figure, a combination of some components, or a different arrangement of the components. For example, the ear-side wearing apparatus may further include a loudspeaker and a camera. Details are not described herein.

Specifically, the processor 1406 may determine, by reading and performing analysis and determining on a measurement signal stored in the memory 1401, whether the measurement electrode can normally perform measurement (whether the ear-side wearing apparatus can normally perform measurement), and perform user attention analysis based on the measurement signal. Details are as follows.

Corresponding to a case of dual-side measurement, the processor 1406 is configured to: determine whether an impedance between the left-ear-side signal measurement unit and the right-ear-side signal measurement unit is less than a preset threshold (a specific determining manner has been described above, and is not described herein again); and when the impedance between the left-ear-side signal measurement unit and the right-ear-side signal measurement unit is less than the preset threshold, obtain the user bioelectrical signal based on a potential difference signal corresponding to a bioelectrical signal collected by the left-ear-side signal measurement unit and a bioelectrical signal collected by the right-ear-side signal measurement unit; or when determining that the ear-side wearing apparatus cannot normally perform measurement (a specific determining method has been described above, and is not described herein again), determine whether an impedance between two of a plurality of left-ear-side signal measurement units is less than the preset threshold and whether an impedance between two of a plurality of right-ear-side signal measurement units is less than the preset threshold, and obtain the user bioelectrical signal based on a potential difference signal corresponding to bioelectrical signals collected by two bioelectrical measurement apparatuses that are on one ear canal side and between which an impedance is less than the preset threshold. The potential difference signal may be obtained by the processor 1406 by executing instructions, or may be obtained by a potential difference obtaining unit, that is, a hardware circuit.

Corresponding to a case of single-side measurement, the processor 1406 is configured to determine whether an impedance between two of single-ear-side signal measurement units is less than a preset threshold (a specific determining manner has been described above, and is not described herein again); and when the impedance between the two single-ear-side signal measurement units is less than the preset threshold, obtain the user bioelectrical signal based on a potential difference signal corresponding to bioelectrical signals collected by the two of the plurality of single-ear-side measurement units.

The processor 1406 is further configured to obtain an attention type of a user based on the electroencephalogram signal. For a specific analysis manner, refer to the foregoing specific embodiment. Details are not described herein again.

It should also be noted that although FIG. 14 is merely an implementation of the ear-side wearing apparatus in this application, in a possible embodiment, the processor 1406 and the memory 1401 in the ear-side wearing apparatus may alternatively be deployed in an integrated manner.

Alternatively, FIG. 14 may show an ear-side wearing apparatus for measuring a user electroencephalogram signal according to an embodiment of the present disclosure. The ear-side wearing apparatus may include one or more processors 1406, one or more memories 1401, an ear-side signal measurement unit 1402, and a characteristic decomposition unit 1403. In specific implementation, the ear-side wearing apparatus may further include a communications unit 1405 (including a sending unit and a receiving unit). The processor 1406 may be connected to all components such as the memory 1401, the measurement electrode 1402, and the characteristic decomposition circuit 1403 by using a bus. The components are separately described as follows.

The processor 1406 is a control center of the ear-side wearing apparatus, and is connected to the components of the ear-side wearing apparatus by using various interfaces and lines. In a possible embodiment, the processor 1406 may further include one or more processing cores. The processor 1400 may determine, by executing program instructions, whether the measurement electrode can normally perform measurement (whether the ear-side wearing apparatus can normally perform measurement). The processor 1406 may be a dedicated processor or a general-purpose processor. When the processor 1406 is a general-purpose processor, the processor 1406 runs or executes software programs (instructions) and/or a module that are/is stored in the memory 1401.

The memory 1401 may include a high-speed random access memory, and may further include a nonvolatile memory, for example, at least one magnetic disk storage device, a flash memory device, or another volatile solid-state storage device. Correspondingly, the memory 1401 may further include a memory controller, to enable the processor 1400 and an input unit to access the memory 1401. The memory 1401 may be specifically configured to store the software programs (instructions) and a collected user bioelectrical signal.

The ear-side signal measurement unit 1402 is configured to collect a user bioelectrical signal from an ear side. Optionally, the ear-side signal measurement unit 1402 may include a left-ear-side signal measurement unit and a right-ear-side signal measurement unit. When the ear-side wearing apparatus 1400 is a single-side measurement apparatus, the ear-side signal measurement unit 1402 may include only a single-ear-side signal measurement unit. The ear-side signal measurement unit 1402 is usually implemented by hardware. For example, the ear-side signal measurement unit 1402 may be an electrode. There may be one or more ear-side signal measurement units 1402.

Optionally, in some embodiments, the ear-side wearing apparatus may further include the characteristic decomposition unit 1403, configured to obtain an electroencephalogram signal from the user bioelectrical signal. The characteristic decomposition unit 1403 is usually implemented by hardware, for example, a characteristic decomposition circuit or an ICA component.

The communications unit 1405 is configured to establish a communication connection to the ear-side wearing apparatus and another device by using a wireless or wired communications technology such as a cellular mobile communications technology, a WLAN technology, or a Bluetooth technology, and send a bioelectrical signal or a collected and processed electroencephalogram signal to a signal analysis apparatus. The signal analysis apparatus in this embodiment of this application may specifically be an attention detection apparatus. The signal analysis apparatus may be the attention detection apparatus. Alternatively, because the obtained electroencephalogram signal may further be used for analysis of other characteristics of the user, for example, recognition of a sleep status and an emotion status, the signal analysis apparatus may be another apparatus that needs to obtain information through analysis of an electroencephalogram signal, for example, a sleep detection apparatus or an emotion detection apparatus.

A person skilled in the art can understand that the ear-side wearing apparatus in this embodiment of this application may include more or fewer components than those shown in the figure, a combination of some components, or a different arrangement of the components. For example, the ear-side wearing apparatus may further include a loudspeaker and a camera. Details are not described herein.

Specifically, the processor 1406 may determine, by reading and performing analysis and determining on a measurement signal stored in the memory 1401, whether the measurement electrode can normally perform measurement (whether the ear-side wearing apparatus can normally perform measurement), and perform user attention type analysis based on the measurement signal. Details are as follows.

Corresponding to a case of dual-side measurement, the processor 1406 is configured to: determine whether an impedance between the left-ear-side signal measurement unit and the right-ear-side signal measurement unit is less than a preset threshold (a specific determining manner has been described above, and is not described herein again); and when the impedance between the left-ear-side signal measurement unit and the right-ear-side signal measurement unit is less than the preset threshold, obtain the user bioelectrical signal based on a potential difference signal corresponding to a bioelectrical signal collected by the left-ear-side signal measurement unit and a bioelectrical signal collected by the right-ear-side signal measurement unit; or when determining that the ear-side wearing apparatus cannot normally perform measurement (a specific determining method has been described above, and is not described herein again), determine whether an impedance between two of a plurality of left-ear-side signal measurement units is less than the preset threshold and whether an impedance between two of a plurality of right-ear-side signal measurement units is less than the preset threshold, and obtain the user bioelectrical signal based on a potential difference signal corresponding to bioelectrical signals collected by two bioelectrical measurement apparatuses that are on one ear canal side and between which an impedance is less than the preset threshold. The potential difference signal may be obtained by the processor 1406 by executing instructions, or may be obtained by a potential difference obtaining unit, that is, a hardware circuit.

Corresponding to a case of single-side measurement, the processor 1406 is configured to determine whether an impedance between two of single-ear-side signal measurement units is less than a preset threshold (a specific determining manner has been described above, and is not described herein again); and when the impedance between the two single-ear-side signal measurement units is less than the preset threshold, obtain the user bioelectrical signal based on a potential difference signal corresponding to bioelectrical signals collected by the two of the plurality of single-ear-side measurement units.

Similarly, FIG. 14 is merely an implementation of the ear-side wearing apparatus in this application, in a possible embodiment, the processor 1406 and the memory 1401 in the ear-side wearing apparatus may alternatively be deployed in an integrated manner.

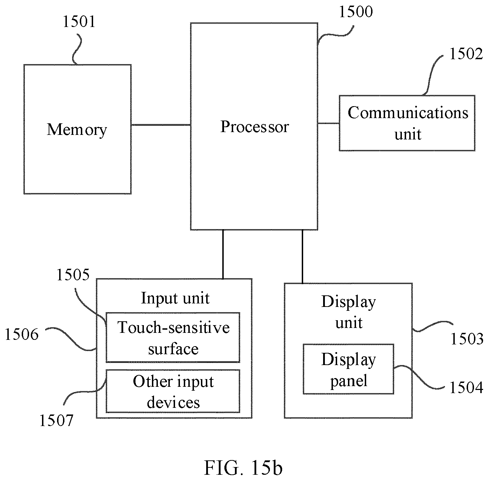

FIG. 15b is a schematic structural diagram of another terminal form of an attention detection apparatus according to an embodiment of this application. As shown in FIG. 15, the attention detection apparatus may include one or more processors 1500 and one or more memories 1501. In specific implementation, the attention detection apparatus may further include components such as an input unit 1506, a display unit 1503, and a communications unit 1502. The processor 2011 may be connected to all components such as the memory 1501, the communications unit 1502, the input unit 1506, and the display unit 1503 by using a bus. The components are separately described as follows.

The processor 1500 is a control center of the attention detection apparatus, and is connected to all the components of the attention detection apparatus by using various interfaces and lines. In a possible embodiment, the processor 1500 may further include one or more processing cores. The processor 1500 may perform attention detection based on an electroencephalogram signal by executing program instructions. The processor 1500 may be a dedicated processor or a general-purpose processor. When the processor 1500 is a general-purpose processor, the processor 1500 runs or executes software programs (instructions) and/or a module that are/is stored in the memory 1501.

The memory 1501 may include a high-speed random access memory, and may further include a nonvolatile memory, for example, at least one magnetic disk storage device, a flash memory device, or another volatile solid-state storage device. Correspondingly, the memory 1501 may further include a memory controller, to enable the processor 1500 and the input unit 1506 to access the memory 1501. The memory 1501 may be specifically configured to store the software programs (instructions) and an electroencephalogram signal.

The input unit 1506 may be configured to receive digital or character information input by a user, and generate keyboard, mouse, joystick, optical, or trackball signal input related to user setting and function control. Specifically, the input unit 1506 may include a touch-sensitive surface 1505 and other input devices 1507. The touch-sensitive surface 1505 is also referred to as a touch display screen or a touch panel, and may collect a touch operation performed by the user on or near the touch-sensitive surface 1505, and drive a corresponding connection apparatus based on a preset program. Specifically, the other input devices 1507 may include but is not limited to one or more of a physical keyboard, a function key, a trackball, a mouse, and a joystick.

The display unit 1503 may be configured to display a search request input by the user or a search result provided by a search apparatus for the user and various graphic user interfaces of the search apparatus, where these graphic user interfaces may include a graphic, a text, an icon, a video, and any combination thereof. Specifically, the display unit 1503 may include a display panel 1504. Optionally, the display panel 1504 may be configured in a form of a liquid crystal display (Liquid Crystal Display, LCD), an organic light-emitting diode (Organic Light-Emitting Diode, OLED), or the like. In FIG. 15, the touch-sensitive surface 1505 and the display panel 1504 are used as two independent components, but in some embodiments, the touch-sensitive surface 1505 and the display panel 1504 may be integrated to implement input and output functions. For example, the touch-sensitive surface 1505 may cover the display panel 1504; and when detecting a touch operation performed on or near the touch-sensitive surface 1505, the touch-sensitive surface 1505 transfers information about the touch operation to the processor 1500 to determine a type of a touch event, and then the processor 1500 provides a corresponding visual output on the display panel 1504 based on the type of the touch event.

The communications unit 1502 is configured to establish a communication connection to an ear-side wearing apparatus and another device by using a wireless or wired communications technology, such as a cellular mobile communications technology, a WLAN technology, or a Bluetooth technology; and receive an electroencephalogram signal sent by the ear-side wearing apparatus, and return an alert signal to the ear-side wearing apparatus based on a determining result, or directly provide an alert by using a loudspeaker, or display an alert interface by using the display unit 1503.

A person skilled in the art can understand that the search apparatus in this embodiment of this application may include more or fewer components than those shown in the figure, a combination of some components, or a different arrangement of the components. For example, the search apparatus may further include a loudspeaker and a camera. Details are not described herein.

Specifically, the processor 1500 may implement, by reading and performing analysis and determining on the electroencephalogram signal stored in the memory 1501, step S103 of detecting an attention type of the user based on the electroencephalogram signal in the embodiments of this application. Step 103 includes:

obtaining sample entropy based on the electroencephalogram signal, where a process of obtaining the sample entropy has been described in detail above, and therefore details are not described herein again;
determining an attention status of the user based on the sample entropy value that is obtained based on the collected electroencephalogram signal; and
determining the attention type of the user based on the sample entropy value and a segmentation value that is obtained by an SVM classifier through machine learning.

For a specific implementation process of a method for performing user attention analysis by the processor 1500, refer to the foregoing method embodiments. Details are not described herein again.

It should also be noted that although FIG. 15b is merely an implementation of the search apparatus in this application, in a possible embodiment, the processor 1500 and the memory 1501 in the search apparatus may alternatively be deployed in an integrated manner.

All or some of the foregoing embodiments may be implemented by software, hardware, firmware, or any combination thereof. When software is used to implement the embodiments, the embodiments may be implemented completely or partially in a form of a computer program product. The computer program product includes one or more computer instructions, and when the computer program instructions are loaded and executed on a computer, all or some of the procedures or functions described in the embodiments of this application are generated. The processor may be a general-purpose processor or a dedicated processor. There may be one or more search apparatuses. When there are a plurality of search apparatuses, the plurality of search apparatuses may form a computer network. The computer instructions may be stored in a computer-readable storage medium or may be transmitted from a computer-readable storage medium to another computer-readable storage medium.

For example, the computer instructions may be transmitted from a website, computer, server, or data center to another website, computer, server, or data center in a wired (for example, a coaxial cable, an optical fiber, or a digital subscriber line) or wireless (for example, infrared and microwave) manner. The computer-readable storage medium may be any usable medium accessible by a computer, or a data storage device, such as a server or a data center, integrating one or more usable media. The usable medium may be a magnetic medium (for example, a floppy disk, a hard disk, or a magnetic tape), an optical medium (for example, a DVD), a semiconductor medium (for example, a solid-state drive), or the like.

For example, an entity for performing the solutions in the embodiments of this application may optionally be an ASIC, an FPGA, a CPU, a GPU, or the like, and the solutions may be implemented by hardware or software. The memory may optionally be a volatile or nonvolatile storage device such as a DDR, an SRAM, an HDD, or an SSD. The data search apparatus may be applied to a plurality of scenarios, for example, applied to a server in a video surveillance system. For example, the data search apparatus may be in a form of a PCIe card.

The ASIC and the FPGA are hardware implementations. To be specific, in hardware design, the methods in this application are implemented by using a hardware description language. The CPU and the GPU are software implementations. To be specific, in software design, the methods in this application are implemented by using software program code.

In the foregoing embodiments, the description of each embodiment has respective focuses. For a part that is not described in detail in an embodiment refer to related descriptions in other embodiments.

The invention is defined by the appended claims.

**Claims**

1. A user attention detection method, wherein the method comprises:

collecting (S101) a user bioelectrical signal from an ear side of a user by using an ear-side wearing apparatus (101, 120, 1100, 1400);
obtaining (S102) a user electroencephalogram signal from the user bioelectrical signal; and
obtaining (S103) an attention type of the user based on the user electroencephalogram signal and a machine learning model; wherein
the ear-side wearing apparatus (101, 120, 1100, 1400) comprises a plurality of ear-side signal measurement units (111, 121, 1402), and the collecting a user bioelectrical signal from the ear side of a user by using an ear-side wearing apparatus (101, 120, 1100, 1400) specifically com-

**EP 3 932 303 B1**

prises:

determining (S211) whether an impedance between two of the ear-side signal measurement units (111, 121, 1402) is less than a preset threshold, collecting bioelectrical signals from the two ear-side signal measurement units (111, 121, 1402) when the impedance between the two ear-side signal measurement units (111, 121, 1402) is less than the preset threshold, obtaining (S212) the user bioelectrical signal based on a potential difference signal corresponding to the bioelectrical signals collected by the two ear-side signal measurement units (111, 121, 1402), and determining, based on an impedance value between ear-side signal measurement units (111, 121, 1402), whether the ear-side signal measurement units (111, 121, 1402) is attached to skin, so as to select different signal collection policies depending on different cases.

2. The method according to claim 1, wherein

the plurality of ear-side signal measurement units (111, 121, 1402) comprise a left-ear-side signal measurement unit (101a, 111a, 121a) and a right-ear-side signal measurement unit (101b, 111b, 121b); and

the determining whether an impedance between two of the ear-side signal measurement units (111, 121, 1402) is less than a preset threshold, collecting bioelectrical signals from the two ear-side signal measurement units (111, 121, 1402) when the impedance between the two ear-side signal measurement units (111, 121, 1402) is less than the preset threshold, and obtaining the user bioelectrical signal based on a potential difference signal corresponding to the bioelectrical signals collected by the two ear-side signal measurement units (111, 121, 1402) is specifically:

determining whether an impedance between the left-ear-side signal measurement unit (101a, 111a, 121a) and the right-ear-side signal measurement unit (101b, 111b, 121b) is less than a preset threshold, and when the impedance between the left-ear-side signal measurement unit (101a, 111a, 121a) and the right-ear-side signal measurement unit (101b, 111b, 121b) is less than the preset threshold, obtaining the user bioelectrical signal based on a potential difference signal corresponding to a bioelectrical signal measured by the left-ear-side signal measurement unit (101a, 111a, 121a) and a bioelectrical signal measured by the right-ear-side signal measurement unit (101b, 111b, 121b).

3. The method according to claim 1, wherein

the ear-side wearing apparatus (101, 120, 1100, 1400) is a single-ear-side wearing apparatus, and the plurality of ear-side signal measurement units (111, 121, 1402) comprise a plurality of single-ear-side signal measurement units (1011); and

the determining whether an impedance between two of the ear-side signal measurement units (111, 121, 1402) is less than a preset threshold, collecting bioelectrical signals from the two ear-side signal measurement units (111, 121, 1402) when the impedance between the two ear-side signal measurement units (111, 121, 1402) is less than the preset threshold, and obtaining the user bioelectrical signal based on a potential difference signal corresponding to the bioelectrical signals collected by the two ear-side signal measurement units (111, 121, 1402) is specifically:

determining whether an impedance between two of the single-ear-side signal measurement units (1011) is less than a preset threshold, and

when the impedance between the two single-ear-side signal measurement units (1011) is less than the preset threshold, obtaining the user bioelectrical signal based on a potential difference signal corresponding to bioelectrical signals collected by the two single-ear-side signal measurement units (1011).

4. The method according to claim 2, wherein the method comprises:

when there are a plurality of left-ear-side signal measurement units (101a, 111a, 121a), and there are a plurality of right-ear-side signal measurement units (101b, 111b, 121b), and when an impedance between one of the left-ear-side signal measurement units (101a, 111a, 121a) and one of the right-ear-side signal measurement units (101b, 111b, 121b) is greater than the preset threshold,

determining whether an impedance between two of the plurality of left-ear-side signal measurement units (101a, 111a, 121a) is less than a preset threshold and whether an impedance between two of the plurality of right-ear-side signal measurement units (101b, 111b, 121b) is less than a preset threshold; and

obtaining the user bioelectrical signal based on a potential difference signal corresponding to bioelectrical signals measured by two bioelectrical measurement apparatuses that are on one ear canal side and between which an impedance is less than the preset threshold.

**5.** The method according to any one of claims 1 to 4, wherein the method comprises:
the obtaining the user bioelectrical signal based on a potential difference signal corresponding to the bioelectrical signals collected by the two ear-side signal measurement units (111, 121, 1402) is specifically: obtaining, by using a differential circuit, the potential difference signal corresponding to the bioelectrical signals collected by the two ear-side signal measurement units (111, 121, 1402), and using the potential difference signal as the user bioelectrical signal.

**6.** The method according to any one of claims 1 to 4, wherein the obtaining an attention type of the user based on the user electroencephalogram signal and a machine learning model is specifically:
calculating a sample entropy value of the user electroencephalogram signal, and analyzing the attention type of the user based on the sample entropy value and the machine learning model.

**7.** The method according to any one of claims 1 to 6, wherein the detecting an attention type of the user based on the user electroencephalogram signal is specifically:

intercepting the user electroencephalogram signal of a preset time length, and obtaining N signal sampling points from the user electroencephalogram signal of the preset time length, wherein
the N signal sampling points are u(1), u(2), ..., and u(N); sequentially intercepting m sampling points based on the N signal sampling points by using each of u(1), u(2), ..., and u(N-m+1) as a start point, to construct N-m+1 m-dimensional vectors; calculating, for each of the N-m+1 m-dimensional vectors, a ratio of a quantity of vectors that are in all the other vectors and whose distances to the m-dimensional vector are less than r to a quantity of all the other vectors, and calculating an average value of the obtained N-m+1 ratios to obtain a first average value; sequentially intercepting m+1 sampling points based on the N signal sampling points by using each of u(1), u(2), ..., and u(N-m) as a start point, to construct N-m (m+1)-dimensional vectors; calculating, for each of the N-m (m+1)-dimensional vectors, a ratio of a quantity of vectors that are in all the other vectors and whose distances to the (m+1)-dimensional vector are less than r to a quantity of all the other vectors, and calculating an average value of the obtained N-m ratios to obtain a second average value; and calculating a sample entropy, SampEn, value based on a ratio of the first average value to the second average value.

**8.** The method according to claim 6 or 7, wherein the machine learning model is an SVM classifier; and machine learning is performed by using the SVM classifier, to obtain a segmentation value, and the attention type of the user is determined based on the segmentation value and the sample entropy value.

**9.** A user attention detection system, wherein the system comprises:

an ear-side wearing apparatus (101, 120, 1100, 1400), configured to: collect (S101) a user bioelectrical signal from an ear side of a user, and obtain (S102) a user electroencephalogram signal from the user bioelectrical signal; and obtain (S103) an attention detection apparatus (102, 130, 1200), configured to detect an attention type of the user based on the user electroencephalogram signal; wherein
that the ear-side wearing apparatus (101, 120, 1100, 1400) comprises a plurality of ear-side signal measurement units (111, 121, 1402), and the ear-side wearing apparatus (101, 120, 1100, 1400) is configured to collect the user bioelectrical signal from the ear side of the user specifically comprises:
the ear-side wearing apparatus determines (S211) whether an impedance between two of the ear-side signal measurement units (111, 121, 1402) is less than a preset threshold, collects bioelectrical signals from the two ear-side signal measurement units (111, 121, 1402) when the impedance between the two ear-side signal measurement units (111, 121, 1402) is less than the preset threshold, obtains (S212) the user bioelectrical signal based on a potential difference signal corresponding to the bioelectrical signals collected by the two ear-side signal measurement units (111, 121, 1402), and determines, based on an impedance value between ear-side signal measurement units (111, 121, 1402), whether the ear-side signal measurement units (111, 121, 1402) is attached to skin, so as to select different signal collection policies depending on different cases.

**10.** The system according to claim 9, wherein

the plurality of ear-side signal measurement units (111, 121, 1402) comprise a left-ear-side signal measurement unit (101a, 111a, 121a) and a right-ear-side signal measurement unit (101b, 111b, 121b); and
that the ear-side wearing apparatus (101, 120, 1100, 1400) determines whether an impedance between two of the ear-side signal measurement units (111, 121, 1402) is less than a preset threshold, collects the bioelectrical signals from the two ear-side signal measurement units (111, 121, 1402) when the impedance between the two ear-side signal measurement units (111, 121, 1402) is less than the preset threshold, and obtains the user bioelectrical signal based on a potential difference signal corresponding to the bioelectrical signals collected by the two ear-side signal measurement units (111, 121, 1402) is specifically:

the ear-side wearing apparatus (101, 120, 1100, 1400) determines whether an impedance between the left-ear-side signal measurement unit (101a, 111a, 121a) and the right-ear-side signal measurement unit (101b, 111b, 121b) is less than a preset threshold, and when the impedance between the left-ear-side signal measurement unit (101a, 111a, 121a) and the right-ear-side signal measurement unit (101b, 111b, 121b) is less than the preset threshold, obtains the user bioelectrical signal based on a potential difference signal corresponding to a bioelectrical signal measured by the left-ear-side signal measurement unit (101a, 111a, 121a) and a bioelectrical signal measured by the right-ear-side signal measurement unit (101b, 111b, 121b).

11. The system according to claim 9, wherein

the ear-side wearing apparatus (101, 120, 1100, 1400) is a single-ear-side wearing apparatus, and the single-ear-side wearing apparatus comprises a plurality of single-ear-side signal measurement units (1011); and

that the ear-side wearing apparatus (101, 120, 1100, 1400) determines whether an impedance between two of the ear-side signal measurement units (111, 121, 1402) is less than a preset threshold, collects bioelectrical signals from the two ear-side signal measurement units (111, 121, 1402) when the impedance between the two ear-side signal measurement units (111, 121, 1402) is less than the preset threshold, and obtains the user bioelectrical signal based on a potential difference signal corresponding to the bioelectrical signals collected by the two ear-side signal measurement units (111, 121, 1402) is specifically:

the ear-side wearing apparatus (101, 120, 1100, 1400) determines whether an impedance between two of the single-ear-side signal measurement units (1011) is less than a preset threshold, and when the impedance between the two single-ear-side signal measurement units (1011) is less than the preset threshold, obtains the user bioelectrical signal based on a potential difference signal corresponding to bioelectrical signals collected by the two single-ear-side signal measurement units (1011).

12. The system according to claim 10, wherein

there are a plurality of left-ear-side signal measurement units (101a, 111a, 121a);
there are a plurality of right-ear-side signal measurement units (101b, 111b, 121b); and
when an impedance between one of the left-ear-side signal measurement units (101a, 111a, 121a) and one of the right-ear-side signal measurement units (101b, 111b, 121b) is greater than the preset threshold,
the ear-side wearing apparatus (101, 120, 1100, 1400) determines whether an impedance between two of the plurality of left-ear-side signal measurement units (101a, 111a, 121a) is less than a preset threshold and whether an impedance between two of the plurality of right-ear-side signal measurement units (101b, 111b, 121b) is less than a preset threshold, and obtains the user bioelectrical signal based on a potential difference signal corresponding to bioelectrical signals measured by two bioelectrical measurement apparatuses that are on one ear canal side and between which an impedance is less than the preset threshold.

13. An ear-side wearing apparatus (101, 120, 1100, 1400), wherein the apparatus comprises:

a plurality of ear-side signal measurement units (111, 121, 1402), configured to collect (S101) a user bioelectrical signal from an ear side;
a first determining unit (114, 124), configured to: determine whether an impedance between two of the ear-side signal measurement units (111, 121, 1402) is less than a preset threshold, and when the impedance between the two ear-side signal measurement units (111, 121, 1402) is less than the preset threshold, use a potential difference signal corresponding to bioelectrical signals collected and measured by the two ear-side signal measurement units (111, 121, 1402) as the user bioelectrical signal, and further determine, based on an impedance value between ear-side signal measurement units (111, 121, 1402), whether the ear-side signal measurement units (111, 121, 1402) is attached to skin, so as to select different signal collection policies depending on different case;
a characteristic decomposition unit (112, 122, 1403), configured to obtain (S102) an electroencephalogram signal from the user bioelectrical signal; and
an attention detection unit (113, 132), configured to obtain (S103) an attention type of a user based on the electroencephalogram signal and a machine learning model.

**14.** The apparatus according to claim 13, wherein

the plurality of ear-side signal measurement units (111, 121, 1402) comprise a left-ear-side signal measurement unit (101a, 111a, 121a) and a right-ear-side signal measurement unit (101b, 111b, 121b); and
the first determining unit (114, 124) is configured to: determine whether an impedance between the left-ear-side signal measurement unit (101a, 111a, 121a) and the right-ear-side signal measurement unit (101b, 111b, 121b) is less than a preset threshold, and when the impedance between the left-ear-side signal measurement unit (101a, 111a, 121a) and the right-ear-side signal measurement unit (101b, 111b, 121b) is less than the preset threshold, obtain the user bioelectrical signal based on a potential difference signal corresponding to a bioelectrical signal measured by the left-ear-side signal measurement unit (101a, 111a, 121a) and a bioelectrical signal measured by the right-ear-side signal measurement unit (101b, 111b, 121b).

**15.** The apparatus according to claim 13, wherein

the ear-side wearing apparatus (101, 120, 1100, 1400) is a single-ear-side wearing apparatus;
the plurality of ear-side signal measurement units (111, 121, 1402) comprise a plurality of single-ear-side signal measurement units (1011); and
that the first determining unit (114, 124) is configured to: determine whether an impedance between two of the ear-side signal measurement units (111, 121, 1402) is less than a preset threshold, and when the impedance between the two ear-side signal measurement units (111, 121, 1402) is less than the preset threshold, use a potential difference signal corresponding to bioelectrical signals collected and measured by the two ear-side signal measurement units (111, 121, 1402) as the user bioelectrical signal is specifically:
the first determining unit (114, 124) is configured to: determine whether an impedance between two of the single-ear-side signal measurement units (1011) is less than a preset threshold, and when the impedance between the two single-ear-side signal measurement units (1011) is less than the preset threshold, use a potential difference signal corresponding to bioelectrical signals collected by the two single-ear-side signal measurement units (1011) as the user bioelectrical signal.

**Patentansprüche**

**1.** Benutzeraufmerksamkeitserkennungsverfahren, wobei das Verfahren Folgendes umfasst:

Erfassen (S101) eines bioelektrischen Benutzersignals von einer Ohrseite eines Benutzers durch Verwenden einer ohrseitigen Tragevorrichtung (101, 120, 1100, 1400);
Erhalten (S102) eines Benutzerelektroenzephalogrammsignals aus dem bioelektrischen Benutzersignal; und
Erhalten (S103) eines Aufmerksamkeitstyps des Benutzers basierend auf dem Benutzerelektroenzephalogrammsignal und einem Modell des maschinellen Lernens;
wobei
die ohrseitige Tragevorrichtung (101, 120, 1100, 1400) eine Vielzahl von ohrseitigen Signalmesseinheiten (111, 121, 1402) umfasst und das Erfassen eines bioelektrischen Benutzersignals von der Ohrseite eines Benutzers durch Verwenden einer ohrseitigen Tragevorrichtung (101, 120, 1100, 1400) insbesondere Folgendes umfasst:
Bestimmen (S211), ob eine Impedanz zwischen zwei der ohrseitigen Signalmesseinheiten (111, 121, 1402) kleiner als ein voreingestellter Schwellenwert ist, Erfassen von bioelektrischen Signalen von den zwei ohrseitigen Signalmesseinheiten (111, 121, 1402), wenn die Impedanz zwischen den zwei ohrseitigen Signalmesseinheiten (111, 121, 1402) kleiner als der voreingestellte Schwellenwert ist, Erhalten (S212) des bioelektrischen Benutzersignals basierend auf einem Potentialdifferenzsignal, das den bioelektrischen Signalen, die durch die zwei ohrseitigen Signalmesseinheiten (111, 121, 1402) erfasst werden, entspricht, und Bestimmen, basierend auf einem Impedanzwert zwischen ohrseitigen Signalmesseinheiten (111, 121, 1402), ob die ohrseitigen Signalmesseinheiten (111, 121, 1402) an der Haut angebracht sind, um in Abhängigkeit von unterschiedlichen Fällen unterschiedliche Signalerfassungsrichtlinien auszuwählen.

**2.** Verfahren nach Anspruch 1, wobei

die Vielzahl von ohrseitigen Signalmesseinheiten (111, 121, 1402) eine linksohrseitige Signalmesseinheit (101a, 111a, 121a) und eine rechtsohrseitige Signalmesseinheit (101b, 111b, 121b) umfasst; und
das Bestimmen, ob eine Impedanz zwischen zwei der ohrseitigen Signalmesseinheiten (111, 121, 1402) kleiner als ein voreingestellter Schwellenwert ist, Erfassen von bioelektrischen Signalen von den zwei ohrseitigen Signalmesseinheiten (111, 121, 1402), wenn die Impedanz zwischen den zwei ohrseitigen Signalmesseinheiten (111, 121, 1402) kleiner als der voreingestellte Schwellenwert ist, und Erhalten des bioelektrischen Benutzersignals basierend auf einem Potentialdifferenzsignal, das den bioelektrischen Signalen, die durch die zwei ohrseitigen Signalmesseinheiten (111, 121, 1402) erfasst werden, entspricht, insbesondere Folgendes ist:

Bestimmen, ob eine Impedanz zwischen der linksohrseitigen Signalmesseinheit (101a, 111a, 121a) und der rechtsohrseitigen Signalmesseinheit (101b, 111b, 121b) kleiner als ein voreingestellter Schwellenwert ist, und, wenn die Impedanz zwischen der linksohrseitigen Signalmesseinheit (101a, 111a, 121a) und der rechtsohrseitigen Signalmesseinheit (101b, 111b, 121b) kleiner als der voreingestellte Schwellenwert ist, Erhalten des bioelektrischen Benutzersignals basierend auf einem Potentialdifferenzsignal, das einem bioelektrischen Signal, das durch die linksohrseitige Signalmesseinheit (101a, 111a, 121a) gemessen wird, und einem bioelektrischen Signal, das durch die rechtsohrseitige Signalmesseinheit (101b, 111b, 121b) gemessen wird, entspricht.

3. Verfahren nach Anspruch 1, wobei

die ohrseitige Tragevorrichtung (101, 120, 1100, 1400) eine einohrseitige Tragevorrichtung ist und die Vielzahl von ohrseitigen Signalmesseinheiten (111, 121, 1402) eine Vielzahl von einohrseitigen Signalmesseinheiten (1011) umfasst; und

das Bestimmen, ob eine Impedanz zwischen zwei der ohrseitigen Signalmesseinheiten (111, 121, 1402) kleiner als ein voreingestellter Schwellenwert ist, Erfassen von bioelektrischen Signalen von den zwei ohrseitigen Signalmesseinheiten (111, 121, 1402), wenn die Impedanz zwischen den zwei ohrseitigen Signalmesseinheiten (111, 121, 1402) kleiner als der voreingestellte Schwellenwert ist, und Erhalten des bioelektrischen Benutzersignals basierend auf einem Potentialdifferenzsignal, das den bioelektrischen Signalen, die durch die zwei ohrseitigen Signalmesseinheiten (111, 121, 1402) erfasst werden, entspricht, insbesondere Folgendes ist:

Bestimmen, ob eine Impedanz zwischen zwei der einohrseitigen Signalmesseinheiten (1011) kleiner als ein voreingestellter Schwellenwert ist, und wenn die Impedanz zwischen den zwei einohrseitigen Signalmesseinheiten (1011) kleiner als der voreingestellte Schwellenwert ist, Erhalten des bioelektrischen Benutzersignals basierend auf einem Potentialdifferenzsignal, das den bioelektrischen Signalen, die durch die zwei einohrseitigen Signalmesseinheiten (1011) erfasst werden, entspricht.

4. Verfahren nach Anspruch 2, wobei das Verfahren Folgendes umfasst:

wenn eine Vielzahl von linksohrseitigen Signalmesseinheiten (101a, 111a, 121a) vorhanden ist, und

eine Vielzahl von rechtsohrseitigen Signalmesseinheiten (101b, 111b, 121b) vorhanden ist, und

wenn eine Impedanz zwischen einer der linksohrseitigen Signalmesseinheiten (101a, 111a, 121a) und einer der rechtsohrseitigen Signalmesseinheiten (101b, 111b, 121b) größer als der voreingestellte Schwellenwert ist,

Bestimmen, ob eine Impedanz zwischen zwei der Vielzahl von linksohrseitigen Signalmesseinheiten (101a, 111a, 121a) kleiner als ein voreingestellter Schwellenwert ist und ob eine Impedanz zwischen zwei der Vielzahl von rechtsohrseitigen Signalmesseinheiten (101b, 111b, 121b) kleiner als ein voreingestellter Schwellenwert ist; und

Erhalten des bioelektrischen Benutzersignals basierend auf einem Potentialdifferenzsignal, das bioelektrischen Signalen entspricht, die durch zwei bioelektrische Messvorrichtungen gemessen werden, die auf einer Seite des Gehörgangs vorhanden sind und zwischen denen eine Impedanz kleiner als der voreingestellte Schwellenwert ist.

5. Verfahren nach einem der Ansprüche 1 bis 4, wobei das Verfahren Folgendes umfasst:

das Erhalten des bioelektrischen Benutzersignals basierend auf einem Potentialdifferenzsignal, das den bioelektrischen Signalen, die durch die zwei ohrseitigen Signalmesseinheiten (111, 121, 1402) erfasst werden, entspricht, insbesondere Folgendes ist:
Erhalten, durch Verwenden einer Differentialschaltung, des Potentialdifferenzsignals, das den bioelektrischen Signalen, die durch die zwei ohrseitigen Signalmesseinheiten (111, 121, 1402) erfasst werden, entspricht, und Verwenden des Potentialdifferenzsignals als das bioelektrische Benutzersignal.

**6.** Verfahren nach einem der Ansprüche 1 bis 4, wobei das Erhalten eines Aufmerksamkeitstyps des Benutzers basierend auf dem Benutzerelektroenzephalogrammsignal und einem Modell des maschinellen Lernens insbesondere Folgendes ist:

Berechnen eines Abtastungsentropiewerts des Benutzerelektroenzephalogrammsignals und Analysieren des Aufmerksamkeitstyps des Benutzers basierend auf dem Abtastungsentropiewert und dem Modell des maschinellen Lernens.

**7.** Verfahren nach einem der Ansprüche 1 bis 6, wobei das Erkennen eines Aufmerksamkeitstyps des Benutzers basierend auf dem Benutzerelektroenzephalogrammsignal insbesondere Folgendes ist:

Abfangen des Benutzerelektroenzephalogrammsignals einer voreingestellten Zeitdauer und Erhalten von N Signalabtastpunkten aus dem Benutzerelektroenzephalogrammsignal der voreingestellten Zeitdauer, wobei die N Signalabtastpunkte u(1), u(2), ... und u(N) sind; sequenzielles Abfangen von m Abtastpunkten basierend auf den N Signalabtastpunkten durch Verwenden von jeweils u(1), u(2), ... und u(N-m+1) als einen Startpunkt, um N-m+1 m-dimensionale Vektoren zu konstruieren; Berechnen, für jeden der N-m+1 m-dimensionalen Vektoren, eines Verhältnisses einer Menge von Vektoren, die in allen der anderen Vektoren vorhanden sind und deren Abstände zu dem m-dimensionalen Vektor kleiner als r sind, zu einer Menge aller der anderen Vektoren, und Berechnen eines Durchschnittswerts der erhaltenen N-m+1 Verhältnisse, um einen ersten Durchschnittswert zu erhalten;
sequenzielles Abfangen von m+1 Abtastpunkten basierend auf den N Signalabtastpunkten durch Verwenden von jeweils u(1), u(2), ... und u(N-m) als einen Startpunkt, um N-m (m+1)-dimensionale Vektoren zu konstruieren; Berechnen, für jeden der N-m (m+1)-dimensionalen Vektoren, eines Verhältnisses einer Menge von Vektoren, die in allen der anderen Vektoren vorhanden sind und deren Abstände zu dem (m+1)-dimensionalen Vektor kleiner als r sind, zu einer Menge aller der anderen Vektoren, und Berechnen eines Durchschnittswerts der erhaltenen N-m Verhältnisse, um einen zweiten Durchschnittswert zu erhalten; und Berechnen eines Abtastentropie(SampEn)-Werts basierend auf einem Verhältnis des ersten Durchschnittswerts zu dem zweiten Durchschnittswert.

**8.** Verfahren nach Anspruch 6 oder 7, wobei das Modell des maschinellen Lernens ein SVM-Klassifikator ist; und

maschinelles Lernen durch Verwenden des SVM-Klassifikators durchgeführt wird, um einen Segmentierungswert zu erhalten, und der Aufmerksamkeitstyp des Benutzers basierend auf dem Segmentierungswert und dem Abtastentropiewert bestimmt wird.

**9.** Benutzeraufmerksamkeitserkennungssystem, wobei das System Folgendes umfasst: eine ohrseitige Tragevorrichtung (101, 120, 1100, 1400), die konfiguriert ist zum:

Erfassen (S101) eines bioelektrischen Benutzersignals von einer Ohrseite eines Benutzers und Erhalten (S102) eines Benutzerelektroenzephalogrammsignals aus dem bioelektrischen Benutzersignal; und
Erhalten (S103) einer Aufmerksamkeitserkennungsvorrichtung (102, 130, 1200), die konfiguriert ist, um einen Aufmerksamkeitstyp des Benutzers basierend auf dem Benutzerelektroenzephalogrammsignal zu erkennen; wobei
dass die ohrseitige Tragevorrichtung (101, 120, 1100, 1400) eine Vielzahl von ohrseitigen Signalmesseinheiten (111, 121, 1402) umfasst und die ohrseitige Tragevorrichtung (101, 120, 1100, 1400) konfiguriert ist, um das bioelektrische Benutzersignal von der Ohrseite des Benutzers zu erfassen, insbesondere Folgendes umfasst:

die ohrseitige Tragevorrichtung bestimmt (S211), ob eine Impedanz zwischen zwei der ohrseitigen Signalmesseinheiten (111, 121, 1402) kleiner als ein voreingestellter Schwellenwert ist, erfasst bioelektrische Signale von den zwei ohrseitigen Signalmesseinheiten (111, 121, 1402), wenn die Impedanz zwischen den zwei ohrseitigen Signalmesseinheiten (111, 121, 1402) kleiner als der voreingestellte Schwellenwert ist, erhält (S212) das bioelektrische Benutzersignal basierend auf einem Potentialdifferenzsignal, das den bioelektrischen Signalen, die durch die zwei ohrseitigen Signalmesseinheiten (111, 121, 1402) erfasst werden, entspricht, und
bestimmt, basierend auf einem Impedanzwert zwischen ohrseitigen Signalmesseinheiten (111, 121, 1402), ob die ohrseitigen Signalmesseinheiten (111, 121, 1402) an der Haut angebracht sind, um in Abhängigkeit von unterschiedlichen Fällen unterschiedliche Signalerfassungsrichtlinien auszuwählen.

**10.** System nach Anspruch 9, wobei

die Vielzahl von ohrseitigen Signalmesseinheiten (111, 121, 1402) eine linksohrseitige Signalmesseinheit (101a, 111a, 121a) und eine rechtsohrseitige Signalmesseinheit (101b, 111b, 121b) umfasst; und

dass die ohrseitige Tragevorrichtung (101, 120, 1100, 1400) bestimmt, ob eine Impedanz zwischen zwei der ohrseitigen Signalmesseinheiten (111, 121, 1402) kleiner als ein voreingestellter Schwellenwert ist, die bioelektrischen Signale von den zwei ohrseitigen Signalmesseinheiten (111, 121, 1402) erfasst, wenn die Impedanz zwischen den zwei ohrseitigen Signalmesseinheiten (111, 121, 1402) kleiner als der voreingestellte Schwellenwert ist, und das bioelektrische Benutzersignal basierend auf einem Potentialdifferenzsignal erhält, das den bioelektrischen Signalen, die durch die zwei ohrseitigen Signalmesseinheiten (111, 121, 1402) erfasst werden, entspricht, insbesondere Folgendes ist:

die ohrseitige Tragevorrichtung (101, 120, 1100, 1400) bestimmt, ob eine Impedanz zwischen der linksohrseitigen Signalmesseinheit (101a, 111a, 121a) und der rechtsohrseitigen Signalmesseinheit (101b, 111b, 121b) kleiner als ein voreingestellter Schwellenwert ist, und, wenn die Impedanz zwischen der linksohrseitigen Signalmesseinheit (101a, 111a, 121a) und der rechtsohrseitigen Signalmesseinheit (101b, 111b, 121b) kleiner als der voreingestellte Schwellenwert ist, das bioelektrische Benutzersignal basierend auf einem Potentialdifferenzsignal erhält, das einem bioelektrischen Signal, das durch die linksohrseitige Signalmesseinheit (101a, 111a, 121a) gemessen wird, und einem bioelektrischen Signal, das durch die rechtsohrseitige Signalmesseinheit (101b, 111b, 121b) gemessen wird, entspricht.

11. System nach Anspruch 9, wobei

die ohrseitige Tragevorrichtung (101, 120, 1100, 1400) eine einohrseitige Tragevorrichtung ist und die einohrseitige Tragevorrichtung eine Vielzahl von einohrseitigen Signalmesseinheiten (1011) umfasst; und

dass die ohrseitige Tragevorrichtung (101, 120, 1100, 1400) bestimmt, ob eine Impedanz zwischen zwei der ohrseitigen Signalmesseinheiten (111, 121, 1402) kleiner als ein voreingestellter Schwellenwert ist, bioelektrische Signale von den zwei ohrseitigen Signalmesseinheiten (111, 121, 1402) erfasst, wenn die Impedanz zwischen den zwei ohrseitigen Signalmesseinheiten (111, 121, 1402) kleiner als der voreingestellte Schwellenwert ist, und das bioelektrische Benutzersignal basierend auf einem Potentialdifferenzsignal erhält, das den bioelektrischen Signalen, die durch die zwei ohrseitigen Signalmesseinheiten (111, 121, 1402) erfasst werden, entspricht, insbesondere Folgendes ist:

die ohrseitige Tragevorrichtung (101, 120, 1100, 1400) bestimmt, ob eine Impedanz zwischen zwei der einohrseitigen Signalmesseinheiten (1011) kleiner als ein voreingestellter Schwellenwert ist, und, wenn die Impedanz zwischen den zwei einohrseitigen Signalmesseinheiten (1011) kleiner als der voreingestellte Schwellenwert ist, das bioelektrische Benutzersignal basierend auf einem Potentialdifferenzsignal erhält, das bioelektrischen Signalen, die durch die zwei einohrseitigen Signalmesseinheiten (1011) erfasst werden, entspricht.

12. System nach Anspruch 10, wobei

eine Vielzahl von linksohrseitigen Signalmesseinheiten (101a, 111a, 121a) vorhanden ist; eine Vielzahl von rechtsohrseitigen Signalmesseinheiten (101b, 111b, 121b) vorhanden ist; und

wenn eine Impedanz zwischen einer der linksohrseitigen Signalmesseinheiten (101a, 111a, 121a) und einer der rechtsohrseitigen Signalmesseinheiten (101b, 111b, 121b) größer als der voreingestellte Schwellenwert ist,

die ohrseitige Tragevorrichtung (101, 120, 1100, 1400) bestimmt, ob eine Impedanz zwischen zwei der Vielzahl von linksohrseitigen Signalmesseinheiten (101a, 111a, 121a) kleiner als ein voreingestellter Schwellenwert ist und ob eine Impedanz zwischen zwei der Vielzahl von rechtsohrseitigen Signalmesseinheiten (101b, 111b, 121b) kleiner als ein voreingestellter Schwellenwert ist, und das bioelektrische Benutzersignal basierend auf einem Potentialdifferenzsignal erhält, das bioelektrischen Signalen entspricht, die durch zwei bioelektrische Messvorrichtungen gemessen werden, die auf einer Seite des Gehörgangs vorhanden sind und zwischen denen eine Impedanz kleiner als der voreingestellte Schwellenwert ist.

13. Ohrseitige Tragevorrichtung (101, 120, 1100, 1400), wobei die Vorrichtung Folgendes umfasst:

eine Vielzahl von ohrseitigen Signalmesseinheiten (111, 121, 1402), die konfiguriert sind, um ein bioelektrisches Benutzersignal von einer Ohrseite aus zu erfassen (S101); eine erste Bestimmungseinheit (114, 124), die konfiguriert ist zum: Bestimmen, ob eine Impedanz zwischen zwei der ohrseitigen Signalmesseinheiten (111, 121, 1402) kleiner als ein voreingestellter Schwellenwert ist, und, wenn die Impedanz zwischen den zwei ohrseitigen Signalmesseinheiten (111, 121, 1402) kleiner als der voreingestellte Schwellenwert ist, Verwenden eines Potentialdifferenzsignals, das bioelektrischen Signalen entspricht, die durch die zwei ohrseitigen Signalmesseinheiten (111, 121, 1402) erfasst und gemessen werden, als das bioelektrische Benutzersignal, und ferner Bestimmen, basierend auf einem Impedanzwert zwischen ohrseitigen Signalmesseinheiten (111, 121, 1402), ob die ohrseitigen Signalmesseinheiten (111, 121, 1402) an der Haut angebracht sind, um in Abhängigkeit von unterschiedlichen Fällen unterschiedliche Signalerfassungsrichtlinien auszuwählen;

eine Eigenschaftzerlegungseinheit (112, 122, 1403), die konfiguriert ist, um aus dem bioelektrischen Benutzersignal ein Elektroenzephalogrammsignal zu erhalten (S102); und

eine Aufmerksamkeitserkennungseinheit (113, 132), die konfiguriert ist, um einen Aufmerksamkeitstyp eines Benutzers basierend auf dem Elektroenzephalogrammsignal und einem Modell des maschinellen Lernens zu erhalten (S103).

**14.** Vorrichtung nach Anspruch 13, wobei

die Vielzahl von ohrseitigen Signalmesseinheiten (111, 121, 1402) eine linksohrseitige Signalmesseinheit (101a, 111a, 121a) und eine rechtsohrseitige Signalmesseinheit (101b, 111b, 121b) umfasst; und

die erste Bestimmungseinheit (114, 124) konfiguriert ist zum: Bestimmen, ob eine Impedanz zwischen der linksohrseitigen Signalmesseinheit (101a, 111a, 121a) und der rechtsohrseitigen Signalmesseinheit (101b, 111b, 121b) kleiner als ein voreingestellter Schwellenwert ist, und, wenn die Impedanz zwischen der linksohrseitigen Signalmesseinheit (101a, 111a, 121a) und der rechtsohrseitigen Signalmesseinheit (101b, 111b, 121b) kleiner als der voreingestellte Schwellenwert ist, Erhalten des bioelektrischen Benutzersignals basierend auf einem Potentialdifferenzsignal, das einem bioelektrischen Signal, das durch die linksohrseitige Signalmesseinheit (101a, 111a, 121a) gemessen wird, und einem bioelektrischen Signal, das durch die rechtsohrseitige Signalmesseinheit (101b, 111b, 121b) gemessen wird, entspricht.

**15.** Vorrichtung nach Anspruch 13, wobei

die ohrseitige Tragevorrichtung (101, 120, 1100, 1400) eine einohrseitige Tragevorrichtung ist;
die Vielzahl von ohrseitigen Signalmesseinheiten (111, 121, 1402) eine Vielzahl von einohrseitigen Signalmesseinheiten (1011) umfasst; und

dass die erste Bestimmungseinheit (114, 124) konfiguriert ist zum: Bestimmen, ob eine Impedanz zwischen zwei der ohrseitigen Signalmesseinheiten (111, 121, 1402) kleiner als ein voreingestellter Schwellenwert ist, und, wenn die Impedanz zwischen den zwei ohrseitigen Signalmesseinheiten (111, 121, 1402) kleiner als der voreingestellte Schwellenwert ist, Verwenden eines Potentialdifferenzsignals, das bioelektrischen Signalen entspricht, die durch die zwei ohrseitigen Signalmesseinheiten (111, 121, 1402) erfasst und gemessen werden, als das bioelektrische Benutzersignal insbesondere Folgendes ist:

die erste Bestimmungseinheit (114, 124) konfiguriert ist zum: Bestimmen, ob eine Impedanz zwischen zwei der einohrseitigen Signalmesseinheiten (1011) kleiner als ein voreingestellter Schwellenwert ist, und, wenn die Impedanz zwischen den zwei einohrseitigen Signalmesseinheiten (1011) kleiner als der voreingestellte Schwellenwert ist, Verwenden eines Potentialdifferenzsignals, das bioelektrischen Signalen entspricht, die durch die zwei einohrseitigen Signalmesseinheiten (1011) erfasst werden, als das bioelektrische Benutzersignal.

**Revendications**

**1.** Procédé de détection d'attention d'utilisateur, dans lequel le procédé comprend :

la collecte (S101) d'un signal bioélectrique d'utilisateur à partir d'un côté oreille d'un utilisateur à l'aide d'un appareil porté côté oreille (101, 120, 1100, 1400) ;
l'obtention (S102) d'un signal d'électroencéphalogramme d'utilisateur à partir du signal bioélectrique d'utilisateur ; et
l'obtention (S103) d'un type d'attention de l'utilisateur sur la base du signal d'électroencéphalogramme d'utilisateur et d'un modèle d'apprentissage automatique ;
dans lequel
l'appareil porté côté oreille (101, 120, 1100, 1400) comprend une pluralité d'unités de mesure de signaux côté oreille (111, 121, 1402), et la collecte d'un signal bioélectrique d'utilisateur à partir du côté oreille d'un utilisateur à l'aide d'un appareil porté côté oreille (101, 120, 1100, 1400) comprend en particulier :
le fait de déterminer (S211) si une impédance entre deux des unités de mesure de signaux côté oreille (111, 121, 1402) est inférieure à un seuil prédéfini, la collecte de signaux bioélectriques à partir des deux unités de mesure de signaux côté oreille (111, 121, 1402) lorsque l'impédance entre les deux unités de mesure de signaux côté oreille (111, 121, 1402) est inférieure au seuil prédéfini, l'obtention (S212) du signal bioélectrique d'utilisateur sur la base d'un signal de différence de potentiel correspondant aux signaux bioélectriques collectés par les deux unités de mesure de signaux côté oreille (111, 121, 1402), et le fait de déterminer, sur la base d'une valeur d'impédance entre des unités de mesure de signaux côté oreille (111, 121, 1402), si les unités de mesure de signaux côté oreille (111, 121, 1402) sont fixées à la peau, de manière à sélectionner différentes politiques de collecte de signaux en fonction de différents cas.

**2.** Procédé selon la revendication 1, dans lequel

la pluralité d'unités de mesure de signaux côté oreille (111, 121, 1402) comprend une unité de mesure de signal côté oreille gauche (101a, 111a, 121a) et une unité de mesure de signal côté oreille droite (101b, 111b, 121b) ; et le fait de déterminer si l'impédance entre deux des unités de mesure de signaux côté oreille (111, 121, 1402) est inférieure à un seuil prédéfini, la collecte de signaux bioélectriques à partir des deux unités de mesure de signaux côté oreille (111, 121, 1402) lorsque l'impédance entre les deux unités de mesure de signaux côté oreille (111, 121, 1402) est inférieure au seuil prédéfini, et l'obtention du signal bioélectrique d'utilisateur sur la base d'un signal de différence de potentiel correspondant aux signaux bioélectriques collectés par les deux unités de mesure de signaux côté oreille (111, 121, 1402) est en particulier :
le fait de déterminer si une impédance entre l'unité de mesure de signal côté oreille gauche (101a, 111a, 121a) et l'unité de mesure de signal côté oreille droite (101b, 111b, 121b) est inférieure à un seuil prédéfini, et lorsque l'impédance entre l'unité de mesure de signal côté oreille gauche (101a, 111a, 121a) et l'unité de mesure de signal côté oreille droite (101b, 111b, 121b) est inférieure au seuil prédéfini, l'obtention du signal bioélectrique d'utilisateur sur la base d'un signal de différence de potentiel correspondant à un signal bioélectrique mesuré par l'unité de mesure de signal côté oreille gauche (101a, 111a, 121a) et à un signal bioélectrique mesuré par l'unité de mesure de signal côté oreille droite (101b, 111b, 121b).

**3.** Procédé selon la revendication 1, dans lequel l'appareil porté côté oreille (101, 120, 1100, 1400) est un appareil porté côté oreille unique, et la pluralité d'unités de mesure de signaux côté oreille (111, 121, 1402) comprend une pluralité d'unités de mesure de signaux côté oreille unique (1011) ; et le fait de déterminer si l'impédance entre deux des unités de mesure de signaux côté oreille (111, 121, 1402) est inférieure à un seuil prédéfini, la collecte de signaux bioélectriques à partir des deux unités de mesure de signaux côté oreille (111, 121, 1402) lorsque l'impédance entre les deux unités de mesure de signaux côté oreille (111, 121, 1402) est inférieure au seuil prédéfini, et l'obtention du signal bioélectrique d'utilisateur sur la base d'un signal de différence de potentiel correspondant aux signaux bioélectriques collectés par les deux unités de mesure de signaux côté oreille (111, 121, 1402) est en particulier :

le fait de déterminer si une impédance entre deux des unités de mesure de signaux côté oreille unique (1011) est inférieure à un seuil prédéfini, et
lorsque l'impédance entre les deux unités de mesure de signaux côté oreille unique (1011) est inférieure au seuil prédéfini,
l'obtention du signal bioélectrique d'utilisateur sur la base d'un signal de différence de potentiel correspondant à des signaux bioélectriques collectés par les deux unités de mesure de signaux côté oreille unique (1011).

**4.** Procédé selon la revendication 2, dans lequel le procédé comprend :

lorsqu'il existe une pluralité d'unités de mesure de signaux côté oreille gauche (101a, 111a, 121a), et
qu'il existe une pluralité d'unités de mesure de signaux côté oreille droite (101b, 111b, 121b), et
lorsqu'une impédance entre l'une des unités de mesure de signaux côté oreille gauche (101a, 111a, 121a) et l'une des unités de mesure de signaux côté oreille droite (101b, 111b, 121b) est supérieure au seuil prédéfini,
le fait de déterminer si une impédance entre deux des unités de mesure de signaux côté oreille gauche (101a, 111a, 121a) est inférieure à un seuil prédéfini et si une impédance entre deux des unités de mesure de signaux côté oreille droite (101b, 111b, 121b) est inférieure à un seuil prédéfini ; et
l'obtention du signal bioélectrique d'utilisateur sur la base d'un signal de différence de potentiel correspondant aux signaux bioélectriques mesurés par deux appareils de mesure bioélectrique situés sur un côté du conduit auditif externe et entre lesquels l'impédance est inférieure au seuil prédéfini.

**5.** Procédé selon l'une quelconque des revendications 1 à 4, dans lequel le procédé comprend :
l'obtention du signal bioélectrique d'utilisateur sur la base d'un signal de différence de potentiel correspondant aux signaux bioélectriques collectés par les deux unités de mesure de signaux côté oreille (111, 121, 1402) est en particulier :
l'obtention, à l'aide d'un circuit différentiel, du signal de différence de potentiel correspondant aux signaux bioélectriques collectés par les deux unités de mesure de signaux côté oreille (111, 121, 1402), et l'utilisation du signal de différence de potentiel comme signal bioélectrique d'utilisateur.

**6.** Procédé selon l'une quelconque des revendications 1 à 4, dans lequel l'obtention d'un type d'attention de l'utilisateur sur la base du signal d'électroencéphalogramme d'utilisateur et d'un modèle d'apprentissage automatique est en particulier :
le calcul d'une valeur d'entropie d'échantillon du signal d'électroencéphalogramme d'utilisateur, et l'analyse du type d'attention de l'utilisateur sur la base de la valeur d'entropie d'échantillon et du modèle d'apprentissage automatique.

**7.** Procédé selon l'une quelconque des revendications 1 à 6, dans lequel la détection d'un type d'attention de l'utilisateur sur la base du signal d'électroencéphalogramme d'utilisateur est en particulier :

l'interception du signal d'électroencéphalogramme d'utilisateur d'une durée prédéfinie, et l'obtention de N points d'échantillonnage du signal d'électroencéphalogramme d'utilisateur de la durée prédéfinie, dans lequel les N points d'échantillonnage de signal sont u(1), u(2), ..., et u(N) ; l'interception séquentielle de m points d'échantillonnage sur la base des N points d'échantillonnage de signal à l'aide de chacun de u(1), u(2), ..., et u(N-m+1) comme point de départ, pour construire N-m+1 vecteurs m-dimensionnels ; le calcul, pour chacun des N-m+1 vecteurs m-dimensionnels, d'un rapport entre une quantité de vecteurs qui sont dans tous les autres vecteurs et dont les distances au vecteur m-dimensionnel sont inférieures à r et une quantité de tous les autres vecteurs, et le calcul d'une valeur moyenne des N-m+1 rapports obtenus pour obtenir une première valeur moyenne ;
l'interception séquentielle de m+1 points d'échantillonnage sur la base des N points d'échantillonnage du signal à l'aide de chacun de u(1), u(2), ..., et u(N-m) comme point de départ, pour N-m vecteurs (m+1)-dimensionnels ;
le calcul, pour chacun des N-m vecteurs (m+1)-dimensionnels, d'un rapport entre une quantité de vecteurs qui sont dans tous les autres vecteurs et dont les distances au vecteur (m+1)-dimensionnel sont inférieures à r et une quantité de tous les autres vecteurs, et le calcul d'une valeur moyenne des N-m rapports obtenus pour obtenir une seconde valeur moyenne ;
et le calcul d'une valeur d'entropie d'échantillon, SampEn, sur la base d'un rapport entre la première valeur moyenne et la seconde valeur moyenne.

**8.** Procédé selon la revendication 6 ou 7, dans lequel le modèle d'apprentissage automatique est un classificateur SVM ; et
l'apprentissage automatique est réalisé à l'aide du classificateur SVM, pour obtenir une valeur de segmentation, et le type d'attention de l'utilisateur est déterminé sur la base de la valeur de segmentation et de la valeur d'entropie d'échantillon.

**9.** Système de détection d'attention d'utilisateur, dans lequel le système comprend :

un appareil porté côté oreille (101, 120, 1100, 1400), configuré pour : collecter (S101) un signal bioélectrique d'utilisateur à partir d'un côté d'oreille d'un utilisateur, et obtenir (S102) un signal d'électroencéphalogramme d'utilisateur à partir du signal bioélectrique d'utilisateur ; et obtenir (S103) un appareil de détection d'attention (102, 130, 1200), configuré pour détecter un type d'attention de l'utilisateur sur la base du signal d'électroencéphalogramme d'utilisateur ; dans lequel
en ce que l'appareil porté côté oreille (101, 120, 1100, 1400) comprend une pluralité d'unités de mesure de signaux côté oreille (111, 121, 1402), et l'appareil porté côté oreille (101, 120, 1100, 1400) configuré pour collecter le signal bioélectrique d'utilisateur à partir du côté oreille de l'utilisateur comprend en particulier :
l'appareil porté côté oreille détermine (S211) si une impédance entre deux des unités de mesure de signaux côté oreille (111, 121, 1402) est inférieure à un seuil prédéfini, collecte les signaux bioélectriques à partir des deux unités de mesure de signaux côté oreille (111, 121, 1402) lorsque l'impédance entre les deux unités de mesure de signaux côté oreille (111, 121, 1402) est inférieure au seuil prédéfini, obtient (S212) le signal bioélectrique d'utilisateur sur la base d'un signal de différence de potentiel correspondant aux signaux bioélectriques collectés par les deux unités de mesure de signaux côté oreille (111, 121, 1402), et détermine, sur la base d'une valeur d'impédance entre des unités de mesure de signaux côté oreille (111, 121, 1402), si les unités de mesure de signaux côté oreille (111, 121, 1402) sont fixées à la peau, de manière à sélectionner différentes politiques de collecte de signaux en fonction de différents cas.

10. Système selon la revendication 9, dans lequel

la pluralité d'unités de mesure de signaux côté oreille (111, 121, 1402) comprend une unité de mesure de signal côté oreille gauche (101a, 111a, 121a) et une unité de mesure de signal côté oreille droite (101b, 111b, 121b) ; et l'appareil porté côté oreille (101, 120, 1100, 1400) détermine si une impédance entre deux des unités de mesure de signaux côté oreille (111, 121, 1402) est inférieure à un seuil prédéfini, collecte les signaux bioélectriques à partir des deux unités de mesure de signaux côté oreille (111, 121, 1402) lorsque l'impédance entre les deux unités de mesure de signaux côté oreille (111, 121, 1402) est inférieure au seuil prédéfini, et obtient le signal bioélectrique d'utilisateur sur la base d'un signal de différence de potentiel correspondant aux signaux bioélectriques collectés par les deux unités de mesure de signaux côté oreille (111, 121, 1402) est en particulier :
l'appareil porté côté oreille (101, 120, 1100, 1400) détermine si une impédance entre l'unité de mesure de signal côté oreille gauche (101a, 111a, 121a) et l'unité de mesure de signal côté oreille droite (101b, 111b, 121b) est inférieure à un seuil prédéfini, et lorsque l'impédance entre l'unité de mesure de signal côté oreille gauche (101a, 111a, 121a) et l'unité de mesure de signal côté oreille droite (101b, 111b, 121b) est inférieure au seuil prédéfini, obtient le signal bioélectrique d'utilisateur sur la base d'un signal de différence de potentiel correspondant à un signal bioélectrique mesuré par l'unité de mesure de signal côté oreille gauche (101a, 111a, 121a) et à un signal bioélectrique mesuré par l'unité de mesure de signal côté oreille droite (101b, 111b, 121b).

11. Système selon la revendication 9, dans lequel

l'appareil porté côté oreille (101, 120, 1100, 1400) est un appareil porté côté oreille unique, et l'appareil porté côté oreille unique comprend une pluralité d'unités de mesure de signaux côté oreille unique (1011) ; et
l'appareil porté côté oreille (101, 120, 1100, 1400) détermine si une impédance entre deux des unités de mesure de signaux côté oreille (111, 121, 1402) est inférieure à un seuil prédéfini, collecte des signaux bioélectriques à partir des deux unités de mesure de signaux côté oreille (111, 121, 1402) lorsque l'impédance entre les deux unités de mesure de signaux côté oreille (111, 121, 1402) est inférieure au seuil prédéfini, et obtient le signal bioélectrique d'utilisateur sur la base d'un signal de différence de potentiel correspondant aux signaux bioélectriques collectés par les deux unités de mesure de signaux côté oreille (111, 121, 1402) est en particulier :
l'appareil porté côté oreille (101, 120, 1100, 1400) détermine si une impédance entre deux des unités de mesure de signaux côté oreille unique (1011) est inférieure à un seuil prédéfini et, lorsque l'impédance entre les deux unités de mesure de signaux côté oreille unique (1011) est inférieure au seuil prédéfini, obtient le signal bioélectrique d'utilisateur sur la base d'un signal de différence de potentiel correspondant à des signaux bioélectriques collectés par les deux unités de mesure de signaux côté oreille unique (1011).

12. Système selon la revendication 10, dans lequel

il existe une pluralité d'unités de mesure de signaux côté oreille gauche (101a, 111a, 121a) ;
il existe une pluralité d'unités de mesure de signaux côté oreille droite (101b, 111b, 121b) ; et
lorsqu'une impédance entre l'une des unités de mesure de signaux côté oreille gauche (101a, 111a, 121a) et l'une des unités de mesure de signaux côté oreille droite (101b, 111b, 121b) est supérieure au seuil prédéfini,
l'appareil porté côté oreille (101, 120, 1100, 1400) détermine si une impédance entre deux de la pluralité d'unités de mesure de signaux côté oreille gauche (101a, 111a, 121a) est inférieure à un seuil prédéfini et si une impédance entre deux de la pluralité d'unités de mesure de signaux côté oreille droite (101b, 111b, 121b) est inférieure à un seuil prédéfini, et obtient le signal bioélectrique d'utilisateur sur la base d'un signal de différence de potentiel correspondant à des signaux bioélectriques mesurés par deux appareils de mesure bioélectriques situés d'un côté du conduit auditif externe et entre lesquels l'impédance est inférieure au seuil prédéfini.

13. Appareil porté côté oreille (101, 120, 1100, 1400), dans lequel l'appareil comprend :

une pluralité d'unités de mesure de signaux côté oreille (111, 121, 1402), configurées pour collecter (S101) un signal bioélectrique d'utilisateur à partir d'un côté oreille ;
une première unité de détermination (114, 124), configurée pour : déterminer si une impédance entre deux des unités de mesure de signaux côté oreille (111, 121, 1402) est inférieure à un seuil prédéfini, et lorsque l'impédance entre les deux unités de mesure de signaux côté oreille (111, 121, 1402) est inférieure au seuil prédéfini, utiliser un signal de différence de potentiel correspondant à des signaux bioélectriques collectés et mesurés par les deux unités de mesure de signaux côté oreille (111, 121, 1402) comme signal bioélectrique d'utilisateur, et déterminer en outre, sur la base d'une valeur d'impédance entre des unités de mesure de signaux côté oreille (111, 121, 1402), si les unités de mesure de signaux côté oreille (111, 121, 1402) sont fixées à la peau, de manière à sélectionner différentes politiques de collecte de signaux en fonction de différents cas ;

une unité de décomposition de caractéristiques (112, 122, 1403), configurée pour obtenir (S102) un signal d'électroencéphalogramme à partir du signal bioélectrique d'utilisateur ; et

une unité de détection d'attention (113, 132), configurée pour obtenir (S103) un type d'attention d'un utilisateur sur la base du signal d'électroencéphalogramme et d'un modèle d'apprentissage automatique.

14. Appareil selon la revendication 13, dans lequel

la pluralité d'unités de mesure de signaux côté oreille (111, 121, 1402) comprend une unité de mesure de signal côté oreille gauche (101a, 111a, 121a) et une unité de mesure de signal côté oreille droite (101b, 111b, 121b) ; et

la première unité de détermination (114, 124) est configurée pour : déterminer si une impédance entre l'unité de mesure de signal côté oreille gauche (101a, 111a, 121a) et l'unité de mesure de signal côté oreille droite (101b, 111b, 121b) est inférieure à un seuil prédéfini, et lorsque l'impédance entre l'unité de mesure de signal côté oreille gauche (101a, 111a, 121a) et l'unité de mesure de signal côté oreille droite (101b, 111b, 121b) est inférieure au seuil prédéfini, obtenir le signal bioélectrique d'utilisateur sur la base d'un signal de différence de potentiel correspondant à un signal bioélectrique mesuré par l'unité de mesure de signal côté oreille gauche (101a, 111a, 121a) et à un signal bioélectrique mesuré par l'unité de mesure de signal côté oreille droite (101b, 111b, 121b).

15. Appareil selon la revendication 13, dans lequel

l'appareil porté côté oreille (101, 120, 1100, 1400) est un appareil porté côté oreille unique ;
la pluralité d'unités de mesure de signaux côté oreille (111, 121, 1402) comprend une pluralité d'unités de mesure de signaux côté oreille unique (1011) ; et
la première unité de détermination (114, 124) est configurée pour : déterminer si une impédance entre deux des unités de mesure de signaux côté oreille (111, 121, 1402) est inférieure à un seuil prédéfini, et lorsque l'impédance entre les deux unités de mesure de signaux côté oreille (111, 121, 1402) est inférieure au seuil prédéfini, utiliser un signal de différence de potentiel correspondant à des signaux bioélectriques collectés et mesurés par les deux unités de mesure de signaux côté oreille (111, 121, 1402) comme signal bioélectrique d'utilisateur est en particulier :

la première unité de détermination (114, 124) est configurée pour : déterminer si une impédance entre deux des unités de mesure de signaux côté oreille unique (1011) est inférieure à un seuil prédéfini, et lorsque l'impédance entre les deux unités de mesure de signaux côté oreille unique (1011) est inférieure au seuil prédéfini, utiliser un signal de différence de potentiel correspondant à des signaux bioélectriques collectés par les deux unités de mesure de signaux côté oreille unique (1011) comme signal bioélectrique d'utilisateur.

102

FIG. 1

```
┌─────────────────────────────────────┐
│ S101. Collect a user bioelectrical   │
│ signal from an ear side of a user by │
│ using an ear-side wearing apparatus  │
└─────────────────────────────────────┘
                    │
                    ▼
┌─────────────────────────────────────┐
│ S102. Obtain a user electroencephalo-│
│ gram signal from the user            │
│ bioelectrical signal                 │
└─────────────────────────────────────┘
                    │
                    ▼
┌─────────────────────────────────────┐
│ S103. Obtain an attention type of    │
│ the user based on the user           │
│ electroencephalogram signal and a    │
│ machine learning model               │
└─────────────────────────────────────┘
```

FIG. 2a

S201. Determine whether an impedance between a left-ear-side signal measurement unit and a right-ear-side signal measurement unit is less than a preset threshold, to determine whether an ear-side wearing apparatus can normally perform measurement

S202. When it is determined that the ear-side wearing apparatus can normally perform measurement, obtain a user bioelectrical signal based on a potential difference signal corresponding to a bioelectrical signal measured by the left-ear-side signal measurement unit and a bioelectrical signal measured by the right-ear-side signal measurement unit

S203. When a determining result is that the ear-side wearing apparatus cannot normally perform measurement, determine whether an impedance between two of a plurality of left-ear-side signal measurement units is less than a preset threshold and whether an impedance between two of a plurality of right-ear-side signal measurement units is less than a preset threshold, and obtain a user bioelectrical signal based on a potential difference signal corresponding to bioelectrical signals measured by two bioelectrical measurement apparatuses that are on one ear canal side and between which an impedance is less than the preset threshold

FIG. 2b

S211. Determine whether an impedance between two signal measurement units of single-ear-side signal measurement units is less than a preset threshold, to determine whether an in-ear apparatus can normally perform measurement

S212. When it is determined that the in-ear device can normally perform measurement, obtain a user bioelectrical signal based on a potential difference signal corresponding to bioelectrical signals measured by the two of the plurality of single-ear-side signal measurement units

FIG. 2c

Gamma wave

Beta wave

Alpha wave

Theta wave

Delta wave

FIG. 3

S1031. Obtain sample entropy based on an electroencephalogram signal

S1032. Determine an attention status of a user based on the sample entropy value that is obtained based on the collected electroencephalogram signal

FIG. 4

FIG. 5

FIG. 6

FIG. 7

FIG. 8a

FIG. 8b

FIG. 9a

FIG. 9b

Ear-side wearing apparatus 1100 → Attention detection apparatus 1200

FIG. 10

Ear-side wearing apparatus 1100

Single-ear-side signal measurement unit 1011

FIG. 11a

Ear-side wearing apparatus 1100

Left-ear-side signal measurement unit 101a

Right-ear-side signal measurement unit 101b

FIG. 11b

Ear-side wearing apparatus 1100

Ear-side signal measurement unit 111

Left-ear-side signal measurement unit 111a

Right-ear-side signal measurement unit 111b

Single-ear-side signal measurement unit 111c

Characteristic decomposition unit 112

First determining unit 114

Second determining unit 115

Attention detection unit 113

FIG. 11c

Ear-side wearing apparatus 120

Ear-side signal measurement unit 121

Left-ear-side signal measurement
unit 121a

Right-ear-side signal measurement
unit 121b

Single-ear-side signal measurement
unit 121c

Characteristic decomposition
unit 122

First determining unit 124

Second determining unit 125

Sending unit 123

FIG. 11d

301
303
302
304

303A
310
303G
303B
304

FIG. 12

404

401

402

403

FIG. 13

```
┌─────────────────────────────────────────┐
│  Attention detection apparatus 130       │
│                                          │
│    ┌───────────────────────────────┐     │
│    │      Receiving unit 131       │     │
│    └───────────────────────────────┘     │
│                                          │
│    ┌───────────────────────────────┐     │
│    │   Attention detection unit    │     │
│    │            132                │     │
│    └───────────────────────────────┘     │
│                                          │
└─────────────────────────────────────────┘
```

FIG. 14

Ear-side wearing apparatus 1400

Communications unit    1405

1402    Ear-side signal measurement unit    Processor 1406    Characteristic decomposition unit    1403

1401    Memory

FIG. 15a

FIG. 15b

S1601. Collect a user bioelectrical signal from an ear side by using an ear-side wearing apparatus

S1602. Obtain a user electroencephalogram signal from the user bioelectrical signal

S1603. Send the user electroencephalogram signal to a signal analysis apparatus

FIG. 16

S1701. Receive a user electroencephalogram signal from an ear-side wearing apparatus

S1702. Obtain an attention type of a user based on the user electroencephalogram signal and a machine learning model

FIG. 17

**EP 3 932 303 B1**

## REFERENCES CITED IN THE DESCRIPTION

### Patent documents cited in the description

- WO 2016110804 A1

### Non-patent literature cited in the description

- automatic detection of drowsiness using in-ear EEG. **NAKAMURA TAKASHI et al.** INTERNATIONAL JOINT CONFERENCE ON NEURAL NETWORKS (IJCNN). IEEE, 08 July 2018